# EUROPEAN PATENT APPLICATION

(11) **EP 1 082 964 A1**
(43) Date of publication of application: **14.03.2001**
(21) Application number: 99923928.8
(22) Date of filing: 04.06.1999
(51) Int. Cl.: A61K 35/74, A23L 1/30, A23L 1/0562

(54) **LACTIC ACID BACTERIUM-CONTAINING COMPOSITIONS, DRUGS AND FOODS**

(30) Priority: 05.06.1998 JP 17215198; 29.09.1998 JP 27551598; 04.11.1998 JP 31351898; 09.12.1998 JP 34950398
(71) Applicant: Wakamoto Pharmaceutical Co., Ltd., Chuo-ku, Tokyo 103-8330 (JP)
(72) Inventor: ISHIKAWA, Hiroshi, Wakamoto Pharmaceuti. Co., Ltd, Tokyo 103-8330 (JP); SUZUKI, Nobuyuki, Wakamoto Pharmaceuti. Co., Ltd., Tokyo 103-8330 (JP); NAKAYA, Seigo, Wakamoto Pharmaceutical Co., Ltd., Tokyo 103-8330 (JP); HIRATA, Haruhisa, Wakamoto Pharmaceuti. Co., Ltd., Tokyo 103-8330 (JP)
(74) Representative: Hubert, Philippe
(86) International application number: JP9903017
(87) International publication number: WO9964023

(57) **Abstract**

The present invention has for its object to provide a lactic acid bacteria-containing composition which is conducive to an increased pharmacologic action of lactic acid bacteria showing such pharmacologic action in the digestive tract and/or the urogential organ and antagonizes the clearance of those lactic acid bacteria from the digestive tract and/or the urogenital organ.

The present invention, therefore, is directed to a lactic acid bacteria-containing composition comprising lactic acid bacteria showing pharmacologic action in the digestive tract and/or the urogenital organ
wherein the adhesion of said lactic acid bacteria to the digestive tract and/or the urogenital organ is enhanced to potentiate said pharmacologic action.

## Description

### TECHNICAL FIELD

The present invention relates to lactic acid bacteria-containing compositions and to pharmaceuticals and foods comprising the same.

### BACKGROUND ART

Lactic acid bacteria-containing pharmaceutical preparations have long been considered to be effective in the treatment of intestinal troubles and, as use-tested over years, are regarded as quite safe remedies. Meanwhile, it is known that *Lactobacillus acidophilus* and *Streptococcus faecalis*, which are formulated as active ingredients of lactic acid bacteria-based pharmaceutical preparations in the Japanese Pharmaceutical Codex Bifidobacteria and Japanese Pharmaceutical Codex Lactomin are residents of the human bowel in which, as the intestinal bacterium, these bacteria are playing a very important role in the maintenance and promotion of health.

Because of reliability of pharmacologic actions and low risks for side effects, these lactic acid bacteria are under intensive research and development with renewed interest today.

Particularly, it is in the field of antigastritic, antiulcerative and urogenital antiinfective agents that amazing progresses are foreseen in the application of lactic acid bacteria.

As therapeutic drugs for peptic ulcer, H2-receptor antagonists such as cimetidine, ranitidine, famotidine, etc., proton pump inhibitors such as omeprazole, etc., and gastric mucosal protectants have so far been developed and met with laudable therapeutic responses. However, there are cases of repeated recurrence and flare-up of ulcers which have once been thought to have healed completely and it has been discovered that the etiology for such recurrent episodes is *Helicobacter pylori* which is detected in biopsy specimens of the gastric mucosa.

*Helicobacter pylori* is a gram-negative microaerophilic helical rod bacillus infecting the gastric mucosa. It has been suggested that by virtue of its strong unease activity, this microorganism decomposes the urea derived from the host to ammonia to neutralize gastric acid and thereby enable itself to grow in the stomach.

It has by now been acknowledged all over the world that elimination of *Helicobacter pylori* is a new therapeutic approach to peptic ulcers, and up to the present, β-lactams such as penicillins, amoxicillin, ampicillin, etc., macrolides such as erythromycin, clarithromycin, azithromycin, roxithromycin, etc., aminoglycoside antibiotics such as arbekacin, gentamicin, etc., tetracycline antibiotics, metronidazole which is in use as an antiprotozoal agent, and bismuth preparations have been found to be effective in eliminating *Helicobacter pylori* and are actually in use in the United States and European countries.

Furthermore, recently in the therapeutic category of antiulcerative agents such as H2-receptor antagonists, proton pump inhibitors, etc., novel compounds further having antibacterial activity against *Helicobacter pylori* have been developed and some already launched on the market. Thus, for the treatment of gastritis or gastroduodenal ulcer and prevention of recurrence thereof, attempts have been made to eliminate *Helicobacter pylori* with antibiotics and other drugs active against this organism.

However, therapeutics using the conventional antibiotics require not only large doses by far surpassing the usual doses required for ordinary infections but also a long duration of medication which may entail the emergence of new resistant strains, and there has also been a risk for side effects.

In lieu of the treatment with antibiotics for the elimination of *Helicobacter pylori*, a probiotic therapy with lactic acid bacteria, for instance, has been contemplated. Japanese Patent No. 2672247 discloses that a strain of *Lactobacillus acidophilus* is effective in eliminating *Helicobacter pylori* from the intestinal and stomach cells. However, when *Lactobacillus acidophilus* was actually administered to patients, the expected effect could not been obtained.

Japanese Kokai Publication Hei-9-241173 discloses that certain strains of *Lactobacillus salivarius* or *Lactobacillus brevis* have antigastritic, antiulcerative and other activities. These strains are effective to some extent but it is highly likely that, when administered orally, the lactic acid bacteria will be rapidly cleared from the stomach and small intestine resulting in a marked attenuation of the *Helicobacter pylori* -eliminating effect.

The second field in which an amazing advance is expected of the application of lactic acid bacteria is their application for the elimination of enterohemorrhagic *Escherichia coli* (EHEC).

EHEC discovered in 1977 is a pathogenic organism named "EHEC-O157" because the majority of its strains belong to the O157:H7 serogroup.

The infection with this bacterial pathogen is frequently found in outbreaks of mass food poisoning caused by the ingestion of contaminated food or water and such prodromal signs as diarrhea and bellyache are followed by the onset of hemorrhagic colitis accompanied by intense pain in the abdomen and bloody stools. Seriously ill patients present with hemolytic-uremic syndrome, thrombotic thrombocytopenia purpura and encephalopathy as complications, with death ensuing in worst cases. The etiologic factor in this infectious disease is vero toxin (VT) produced by EHEC. VT is classified into two types, namely VT1 which is identical to Shiga toxin produced by *Shigella dysenteriae* and VT2 which resembles VT1 in biological activity but is different in physicochemical and immunological properties is known also as Shiga-like toxin.

VT is the most virulent of all cytotoxins and, owing to the protein synthesis-inhibition induced by its RNA N-glycosidase activity, strong murinolethal activity, cytotoxicity and enterotoxicity are expressed. In EHEC infections, antibiotics such as fosfomycin, norfloxacin, kanamycin, etc. are used but no adequate therapeutic modality has been available to this day and development of a new therapeutic approach is awaited.

The third field in which an amazing advance is expected of the application of lactic acid bacteria is their application for the treatment of food allergy.

The number of food allergy patients is increasing steadily in the world and this trend is particularly remarkable in the advanced countries. For the therapy and/or prophylaxis of food allergies, omission of causative foods and administration of antiallergic drugs have so far been practiced but the omission of foods imposes great physical and mental burdens on individuals and is subject to many limitations from the standpoint of family life or group life. Prolonged administration of antiallergic drugs involves high risks for side effects.

Accounting for the recent increase in the incidence of food allergy in the advanced countries, it has been conjectured that because of the by-now decreased chances for man being exposed to various pathogenic microorganisms, the constitution of human beings has been altered so as to react excessively to such antigenic stimuli. It is known that as a characteristic of the defense mechanism of experimentally constructed germ-free animals, excessive reactions to the invasion of food allergens are shown.

While it is known that bottle-fed infants show a significantly high incidence of allergic disease as compared with breast-fed infants and the same is true of the incidence of hyperIgEemia, a check of fecal colonies reveals that the population of bifidobacteria is significantly low in bottle-fed infants as compared with breast-fed infants and, conversely, the former infants give higher *clostridium* and *pseudomonal* counts or detection rates ["Effects of Feeding on the Fecal Flora of Infants", Mitsuoka, T.: "Intestinal Flora and Nutrition", p. 13, Gakkai Shuppan Center, 1983].

In recent years it has been reported that the onset of allergy is associated with the balance between Th1 and Th2 cells, with a diminished proportion of Th1 cells leading to the onset of allergy [Modern Medicine, 53, No. 12, p. 72-79, 1998],that Th1 cells are induced by IL-12 [Advances in Medicine, 180, p. 85-89, 1997], and that lactic acid bacteria have IL-12 production-stimulating activity [Japanese Kokai Publication Hei-10-139674].

Therefore, it is expected that food allergy may be prevented and cured through the intake of bifidobacteria.

While the new application range of lactic acid bacteria is expanding and assuming more and more importance, the fundamental problem remains that the lactic acid bacteria administered are rapidly cleared from the digestive tract and there is no effective measure to prevent this early clearance and uphold or potentiate their pharmacologic action.

Meanwhile, the fourth field in which an amazing progress is also expected of the application of lactic acid bacteria is their application to the therapy and/or prophylaxis of urogenital organ infections.

Established in the vagina of a normal healthy woman is a well-balanced flora with lactobacilli as dominant members. However, as the balance of this vaginal flora is disturbed by weakening of the self-cleaning action of the vagina, insertion of an intrauterine contraceptive device, chemical or mechanical stimuli, derangement of estrogen function and/or infection associated with a coitus, lactobacilli are replaced by a number of other species of bacteria to cause onset of bacterial vaginosis or mycotic vaginosis. Furthermore, lactobacilli are suspected to form a protective biofilm at the urinary meatus to thereby prevent the ascending migration of gram-negative rod bacteria such as *Escherichia coli* from the perianal region and protecting the urinary meatus against bacterial infections. Thus, the breakdown of this protective biofilm of lactobacilli may lead to recurrent episodes of urinary tract infection.

When such bacterial vaginosis, recurrent urinary tract infection, etc. are left untreated, they may progress to the more serious diseases such as intrapelvic infection, postoperative infection, and it is reported that perinatal infection and patients with bacterial vaginosis are liable to acquire an HIV infection [AIDS, vol. 2, p. 1699-1706, 1998].

In such urogenital infections, a probiotic therapy with lactic acid bacteria has been attempted [The Japanese Journal of Antibiotics, 759 (No. 39), p. 51-12, 1998; FEMS Immunology and Medical Microbiology, 6, 251-264, 1993]. However, such infections of the urogenital organ are characterized in that even if a remission is obtained once, relapses will be experienced and a complete cure is difficult.

While, in this manner, the new application of lactic acid bacteria is broadening and assuming more and more importance, the fundamental problem remains that, after administration, lactic acid bacteria are rapidly cleared from the urogenital organ and there is no effective measure to prevent this early clearance and uphold and/or potentiate their pharmacologic action.

### SUMMARY OF THE INVENTION

In the above state of the art, the present invention has for its object to provide a lactic acid bacteria-containing composition
which is conducive to an increased pharmacologic action of lactic acid bacteria showing such pharmacologic action in the digestive tract and/or the urogential organ and antagonizes the clearance of those lactic acid bacteria from the digestive tract and/or the urogenital organ.

The present invention, therefore, is directed to a lactic acid bacteria-containing composition comprising lactic acid bacteria showing pharmacologic action in the digestive tract and/or the urogenital organ
wherein the adhesion of said lactic acid bacteria to the digestive tract and/or the urogenital organ is enhanced to potentiate said pharmacologic action.

### DETAILED DESCRIPTION OF THE INVENTION

The present invention relates to a lactic acid bacteria-containing composition comprising lactic acid bacteria showing pharmacologic action in the digestive tract and/or the urogenital organ wherein the adhesion of said lactic acid bacteria to the digestive tract and/or the urogenital organ is enhanced to potentiate pharmacologic action.

The inventors of the present invention postulated that in order to antagonize the clearance of a composition containing a lactic acid bacteria showing pharmacologic action from the digestive tract and/or the urogenital organ after ingestion or administration and thereby potentiate its pharmacologic action, it would be instrumental to allow those bacteria for prolonged retention in the digestive tract and/or the urogenital organ and, to meet that objective, to improve the adhesion of the lactic acid bacteria to the digestive tract and/or the urogenital organ. The present invention is accomplished by the finding.

The above series of contrivances namely potentiation of pharmacologic action, prolonged retention in the digestive tract and/or the urogenital organ, and enhanced adhesion to the digestive tract and/or the urogenital organ might appear obvious on paper but it was quite difficult for anyone to even conceive of such ideas on lactic acid bacteria in the state of the art where the adhesion of lactic acid bacteria to the digestive tract and/or the urogenital organ was technically near to impossibility. Only through the acquisition of specific substances having adhesion-improving properties which will be described in detail hereinafter, the present inventors could arrive at the present invention.

The term "digestive tract" as used in this specification is a generic term meaning the canal of the body which is in charge of digestion and absorption and includes the mouth, esophagus, stomach, small intestine, large intestine and rectum. Also as used in this specification, the term "urogenital organ" is a generic term meaning the organ involved in the production and excretion of urine and the organ related to reproduction, thus including the vulva and vagina of women and the genital organ and urinary tract of men. The term "digestive tract and/or the urogenital organ" is used in this specification to mean the digestive tract only, the urogenital organ only, or both the digestive tract and the urogenital organ depending on the context.

The lactic acid bacteria-containing composition in the context of this specification means a composition containing lactic acid bacteria. The adherence of lactic acid bacteria to the digestive tract and/or the urogenital organ in the context of this specification means that lactic acid bacteria continue to be present on the surface of mucosal epithelial cells of the digestive tract and/or of the urogenital organ. Improvement in the adhesion of lactic acid bacteria to the digestive tract and/or the urogenital organ means an increase in the number of lactic acid bacteria which continue to be present on the surface of mucosal epithelial cells of the digestive tract and/or of the urogenital organ. In the present invention, said increase in the number of bacteria (also referred to as the number of adherent bacteria) is at least about 2-fold, usually as great as 10- through 100-fold as compared with the untreated control.

The fact that the pharmacologic action of lactic acid bacteria is enhanced as the result of an improved adhesion thereof to the digestive tract and/or the urogenital organ has been experimentally demonstrated by the present inventors. It is, therefore, obvious that improving the adhesion of lactic acid bacteria to the digestive tract and/or the urogenital organ is a very effective method of enhancing said pharmacologic action. It has also been found that for the purpose of enhancing said pharmacologic action, the lactic acid bacteria to be adhered to the digestive tract and/or the urogenital organ are preferably living organisms.

The lactic acid bacteria-containing composition of the present invention contains lactic acid bacteria. The lactic acid bacteria mentioned above include various bacteria useful for man and animals, thus including:

Lactic acid bacilli such as *Lactobacillus salivarius*, *Lactobacillus acidophilus*, *Lactobacillus brevis, Lactobacillus rhamnosus*, *Lactobacillus plantarum, Lactobacillus helveticus*, *Lactobacillus fermentum*, *Lactobacillus paracasei*, *Lactobacillus casei, Lactobacillus delbrueckii*, *Lactobacillus reuteri*, *Lactobacillus buchneri*, *Lactobacillus gasseri*, *Lactobacillus johnsonii*, *Lactobacillus kefir*, etc.;
lactic acid cocci such as *Lactococcus lactis*, *Lactococcus plantarum*, *Lactococcus raffinolactis*, *Enterococcus faecalis*, *Enterococcus faecium*, *Streptococcus thermophilus*, *Leuconostoc lactis*, *Leuconostoc mesenteroides*, etc.; and
bifidobacteria such as *Bifidobacterium animalis*, *Bifidobacterium bifidum*, *Bifidobacterium breve*, *Bifidobacterium infantis*, *Bifidobacterium longum*, *Bifidobacterium pseudolongum*, *Bifidobacterium thermophilum*, *Bifidobacterium adolescentis*, and so on.

These lactic acid bacteria can be used singly or two or more of them may be used.

As said lactic acid bacteria, those grown by the routine cultural method for lactic acid bacteria and harvested by a separatory procedure such as centrifugation can be used as they are in the present invention.

The present invention is characterized in that the adhesion of said lactic acid bacteria to the digestive tract and/or the urogenital organ is enhanced, therefore any method can be available as long as the adhesion of said lactic acid. bacteria to the digestive tract and/or the urogenital organ can be enhanced, but it is preferred that the method comprises incorporating a macromolecular substance, preferably a basic polymer.

The basic polymer is not particularly restricted, only provided that it is a basic substance having a high molecular weight. Moreover, in case of an amphoteric polymer, polymers, for example a basic protein having an isoelectric point on the alkaline side, which is cationic when the pH is lower than its isoelectric point also falls within the scope of the "basic polymer". Furthermore, the term "polymer" is used in this specification to cover substances like chitosan tetramer having a molecular weight of about 808.

The basic polymer mentioned above includes chitosan, polylysine, antihyperlipidemic anion exchange resins such as cholestyramine resin and colestimide, protamine, gelatin, aminoalkyl methacrylate copolymer E, aminoalkyl methacrylate copolymer RS, lactoferrin and lysozyme, among others,

The chitosan mentioned above is a macromolecular polysaccharide obtainable by chemical treatment of chitin which is a main component of the exoskeleton of a crab, the shell of a shrimp, the cuticle of insects such as a locust, silkworm, chrysalis, etc., the cell wall of fungi of the family. *Basidiomycetes* such as shiitake, enokidake, etc., and fungi of the family *Zygomycetes* such as *Rhizopus species*, etc. These are known to have antitumorigenic, antimicrobial, immunopotentiating and other activities.

Japanese Kokai Publication Hei-8-165246 discloses a physiological activity enhancer comprising a high-nucleic acid substance and chitosan and Japanese Kokai Publication Hei-10-191928 discloses a health food containing chitosan and bifidobacteria. However, it has not been found that chitosan ever contributes to improved adhesion of lactic acid bacteria to the digestive tract and/or the urogenital organ and in view of the effects achieved by the present invention, it appears to have been difficult to anticipate the present invention.

Chitosan for use in the present invention is a deacetylation product obtainable by alkali treatment of native chitin. This chitosan occurs in a broad range of grades according to the order of average molecular weight and the degree of deacetylation, but the chitosan which can be used in the present invention includes any and all of such grades of chitosan (inclusive of chitosan oligosaccharide). As is well known, chitosan is a foodstuff; therefore, it is highly safe and quite suitable for the purposes of the present invention.

The amount of use of chitosan relative to lactic acid bacteria in the present invention is preferably not less than 0.00015 mg, more preferably not less than 0.001 mg, per 1×10⁹ lactic acid bacteria.

Polylysine which can be used in the present invention is a homopolymer of the essential amino acid L-lysine as formed by n × amide linkages between the ε -N terminal and C-terminal of L-lysine and the degree of polymerization (n) may be about 20 to 25. Polylysine is a known compound whose growth inhibitory activity against microorganisms has been already established.

The amount of use of polylysine relative to lactic acid bacteria in the present invention is preferably not less than 0.00015 mg, more preferably not less than 0.001 mg, per 1×10⁹ lactic acid bacteria.

The antihyperlipedemic anion exchange resin which can be used in the present invention is not particularly restricted but includes cholestyramine resin and colestimide, among others. The cholestyramine resin is a known synthetic cholesterol-binding strongly basic ion exchange resin. It is commercially available under the tradenames of Dowex; 1-X2-Cl, MK-135, etc. In Merck Index, too, it is listed under No. 2257. The colestimide mentioned above is a known antihyperlipidemic compound commercially available under the tradename of Cholebine.

The amount of use of the antihyperlipidemic anion exchange resin relative to lactic acid bacteria in the present invention is preferably not less than 0.00015 mg, more preferably not less than 0.001 mg, per 1×10⁹ lactic acid bacteria.

Protamine for use in the present invention is a strongly basic protein occurring principally as the nucleoprotein (nucleoprotamine) bound to DNA in the nuclei of spermatids of fish, and is a known compound as described in, inter alia, J. Antibact. Antifung. Agents Vol. 23, No. 10, pp 635-642 (1995). As of today, protamine is chiefly used in the food industry as a food preservative taking advantage of its antibacterial activity, and its amino acid composition and stereostructure have been elucidated.

The amount of use of protamine relative to lactic acid bacteria in the present invention is preferably not less than 0.00015 mg, more preferably not less than 0.001 mg, per 1×10⁹ lactic acid bacteria.

Gelatin for use in the present invention is a protein which can be prepared by acid or alkali treatment of the animal bone, ligament or tendon and hot-water extraction of the resulting crude collagen, and is a known substance described in, inter alia, The Japanese Pharmacopoeia.

When gelatin is to be used in the practice of the present invention, acid-treated gelatin is preferably used. The isoelectric point of the acid-treated gelatin is pH 7.0 to 9.0 and this gelatin is positively charged when the pH is lower than the isoelectric point and, as such, can be one of the basic polymers in the context of this specification.

The amount of use of gelatin relative to lactic acid bacteria in the present invention is preferably not less than 0.00015 mg, more preferably not less than 0.001 mg, per 1×10⁹ lactic acid bacteria.

Aminoalkyl methacrylate copolymer E for use in the present invention is a terpolymer of methyl methacrylate/butyl methacrylate/and dimethylaminoethyl methacrylate and has been used as a coating agent for pharmaceutical tablets and granules, thus being a known substance. It is available under the tradename of Eudragit E (Röhm), for instance. Aminoalkyl methacrylate copolymer E is soluble in organic solvents but almost insoluble in water but since it exhibits basicity, this substance also belongs to the category of basic polymer so referred to in this specification.

The amount of use of aminoalkyl methacrylate copolymer E relative to lactic acid bacteria in the present invention is preferably not less than 0.00015 mg, more preferably not less than 0.001 mg, per 1×10⁹ lactic acid bacteria.

Aminoalkyl methacrylate copolymer RS for use in the present invention is a terpolymer of ethyl acrylate/methyl methacrylate/chlorotrimethylammoniumethyl methacrylate, being a compound known as a coating agent for pharmaceutical tablets and granules. It is commercially available under the tradenames of Eudragit RS, Eudragit Retard S, and Eudragit RL (Röhm), among others. Aminoalkyl methacrylate copolymer RS is soluble in organic solvents and almost insoluble in water but, because it shows basicity, falls under the category of "basic polymer" so referred to in this specification.

The amount of use of aminoalkyl methacrylate copolymer RS relative to lactic acid bacteria is preferably not less than 0.00015 mg, more preferably not less than 0.001 mg, per 1×10⁹ lactic acid bacteria.

Lactoferrin for use in the present invention is a substance already known as a multifunctional protein occurring in human and animal milks. Lactoferrin is a protein having a molecular weight of about 8×10⁴, and the amino acid sequences of human lactoferrin and bovine lactoferrin have been identified. Since it exhibits basicity, lactoferrin is one of the basic polymer so referred to in this specification.

The amount of use of lactoferrin relative to lactic acid bacteria in the present invention is preferably not less than 0.00015 mg, more preferably not less than 0.001 mg, per 1×10⁹ lactic acid bacteria.

Lysozyme is a substance known as an enzyme which hydrolyzes the β1-4 linkages between the N-acetylmuramic acid and N-acetylglucosamine of the bacterial cell wall mucopeotide (EC3. 2. 1. 17.). For example, the lysozyme derived from the chicken egg white is a single-stranded polypeptide consisting of 129 amino acid residues, with a molecular mass of 14300 and an isoelectric point of 11, thus being one of basic polymers so referred to in this specification.

The amount of use of lysozyme relative to lactic acid bacteria in the present invention is preferably not less than 0.00015 mg, more preferably not less than 0.001 mg, per 1×10⁹ lactic acid bacteria.

In the present invention, the basic polymers mentioned above can be used singly or two or more of them can be used.

The lactic acid bacteria-containing composition can be used for the purpose of eliminating *Helicobacter pylori*. While lactic acid bacteria have an *Helicobacter pylori*-eliminating action as mentioned above, the lactic acid bacteria-containing composition of the present invention, in which the adhesion of said bacteria to the digestive tract and/or the urogenital organ is enhanced, exhibits a by far more potent eliminating effect on *Helicobacter pylori* than the composition exclusively comprising lactic acid bacteria.

The lactic acid bacteria-containing composition of the present invention can be used for the purpose of eliminating enterohemorrhagic *Escherichia coli*. While lactic acid bacteria have an enterohemorrhagic *Escherichia coli*-eliminating action as mentioned above, the lactic acid bacteria-containing composition of the present invention, in which, the adhesion of the bacteria to the digestive tract is enhanced, exhibits a by far superior enterohemorrhagic *Escherichia coli*-eliminating action as compared with the composition exclusively comprising lactic acid bacteria.

The lactic acid bacteria-containing composition of the present invention can be used in the therapy of food allergy. While lactic acid bacteria have been demonstrated to have a therapeutic effect on food allergies as mentioned hereinbefore, the lactic acid bacteria-containing composition, in which the adhesion of those bacteria to the digestive tract is enhanced, exhibits a remarkably improved efficacy in the treatment of food allergies as compared with the composition exclusively comprising lactic acid bacteria.

The lactic acid bacteria-containing composition of the present invention can be used in the therapy and/or prophylaxis of urogenital organ infections. While lactic acid bacteria have been shown to be efficacious in the therapy and/or prophylaxis of infections of the urogenital organ as mentioned hereinbefore, the lactic acid bacteria-containing composition in which the adhesion of those bacteria to the urogenital organ is enhanced, exhibits a by far superior efficacy in the therapy and/or prophylaxis of infections of the urogenital organ as compared with the composition exclusively comprising lactic acid bacteria.

The urogenital organ infections mentioned above are not particularly restricted but include bacterial vaginosis, mycotic vaginosis and recurrent urinary tract infection, among others.

The lactic acid bacteria-containing composition of the present invention can be produced by various methods. The lactic acid bacteria-containing composition of the present invention cart be obtained by, for example, the method which comprises mixing a powder of said basic polymer with a suspension of lactic acid bacteria, the method which comprises mixing an aqueous solution or suspension of said basic polymer with a suspension of lactic acid bacteria, or the method which comprises mixing an aqueous solution or suspension of said basic polymer with a powder of lactic acid bacteria.

Furthermore, the composition can be supplemented with a suitable stabilizer and powderized by drying, for example lyophilization. As the condition of mixing variables, concentrations are not particularly restricted but the temperature and pH are preferably selected judiciously in consideration of the stability of lactic acid bacteria.

For example, the temperature and pH of said suspension of lactic acid bacteria and those of said aqueous solution or suspension of basic polymer are preferably not higher than 60°C and pH 2 to 9, respectively. The lactic acid bacteria-containing, composition of the present invention can also be obtained by mixing a powder of said basic polymer with a powder of lactic acid bacteria. When chitosan or aminoalkyl methacrylate copolymer E is used as the basic polymer to prepare an aqueous solution of chitosan or aminoalkyl methacrylate copolymer E, it is preferable to add an acid component, for generally both chitosan and aminoalkyl methacrylate copolymer E may swell and dissolve under acidic conditions.

The acid component mentioned above is not particularly restricted but includes hydrochloric acid, sulfuric acid, acetic acid, lactic acid, adipic acid, succinic acid, malic acid, citric acid and tartaric acid, among others.

The lactic acid bacteria-containing composition of the present invention, thus obtained, is administered to, or caused to be ingested by, man and animals as a medicine or a food. The medicine comprising the lactic acid bacteria-containing composition of the present invention is also an embodiment of the present invention and the food comprising the lactic acid bacteria-containing composition of the present invention is also another embodiment of the present invention.

The medicine or food mentioned above can be provided in a variety of forms by the conventional technology. As to the dosage form for application to the gastric tract, powders, fine granules, granules, capsules, tablets, troches, syrups, etc. can be safely administered by the oral route. With regard to the dosage form for application to the urogenital organ, suppositories, tablets, capsules, creams, gels, ointments, lotions, washes and douches can be mentioned by way of example.

These pharmaceutical preparations can be manufactured by formulating the lactic acid bacteria-containing composition with the various additives which are in routine use in pharmaceutical or food processing procedures, such as the excipient, binder, disintegrator, coating agent, lubricant, etc., in the known manner.

The dosage in humans is dependent on the target disease and the severity of illness but in the case of an adult patient. not less than 1×10⁶ viable cells, preferably 1×10⁸ to 1×10¹² viable cells, can be administered once or in a few divided doses a day according to need.

The excipient includes sugars such as sucrose, lactose, mannitol, glucose, etc. and starches such as corn starch, potato starch, rice starch, partially pregelatinized starch and so on.

The binder includes polysaccharides such as dextrin, sodiumm alginate, carrageenan, guar gum, acacia, agar, etc., naturally-occurring macromolecular substances such as tragacanth, gelatin, gluten, etc., cellulose derivatives such as hydroxypropylcellulose, methylcellulose, hydroxypropylmethylcellulose, ethylcellulose, hydroxypropylethylcellulose, carboxymethylcellulose sodium, etc., and synthetic polymers such as polyvinylpyrrolidone, polyvinyl alcohol, polyvinyl acetate, polyethylene glycol, polyacrylic acid, polymethacrylic acid, and vinyl acetate resin, among others.

The disintegrator includes cellulose derivatives such as carboxymethylcellulose, carboxymethylcellulose calcium, low substituted hydroxypropylcellulose, etc. and starches such as sodium carboxymethylstarch,hydroxypropylstarch, corn starch, potato starch, rice starch and partially pregelatinized starch, among others.

The lubricant includes talc, stearic acid, calcium stearate, magnesium stearate, colloidal silica, hydrous silicon dioxide, various kinds of waxes and hydrogenated oils, etc.

The coating agent includes water-insoluble polymers such as dimethylaminoethyl methacrylate-methacrylic acid copolymer, polyvinyl acetal diethylaminoacetate, ethyl acrylate-methacrylic acid copolymer, ethyl acrylate-methyl methacrylate-chlorotrimethylammoniumethyl methacrylate copolymer, ethylcellulose, etc., enteric polymers such as methacrylic acid-ethyl acrylate copolymer, hydroxypropylmethylcellulose phthalate, hydroxypropylmethylcellulose acetate succinate, etc., and water-soluble polymers such as methylcellulose, hydroxypropylmethylcellulose, polyvinylpyrrolidone, polyethylene glycol, etc.

The lactic acid bacteria-containing composition of the present invention can be added to foods. The kinds of foods are not particularly restricted but include yogurt and other fermented milk products.

### BEST MODE FOR CARRYING OUT THE INVENTION

The following working, test and formulation examples are intended to illustrate the present invention in further detail and should by no means be construed as defining the scope of the present invention.

In the evaluation of the various effects of the present invention, the human stomach cancer cell line MKN45 (JCRB 0254, Yamaguchi, H. et al: The Journal of the Japanese Association for Infectious Diseases, 72, 487, 1998) was used as the stomach cell model as is routinely used; the human colon cancer cell line Caco 2(ATCC HTB-37, Coconnier M. H. et al.: FEMS Microbiol. Lett., 110, 299, 1993) was used as the intestinal tract cell model as is routinely used; the monocytic leukemia cell line THP-1 which secretes IL-12 in the presence of lactic acid bacteria (Infect. Immun., 64, 1906, 1996) was used as the food allergy model cell; and the human uterine cancer cell line HeLa (RCB007, Gey GO et al.: Cancer Res., 12, 264, 1952) was used as the urogenital organ cell model as is routinely used. These are intended to facilitate evaluations in vitro.

### Example 1

MRS Broth (Difco) was inoculated with lactic acid bacteria and a liquid culture was carried out at 37°C for 16 hours. After completion of culture, the culture fluid was centrifuged at 3000 rpm for 5 minutes to recover a cellular concentrate. To this concentrate was added phosphate-buffered saline adjusted to pH 5 with 1 N-hydrochloric acid in advance (hereinafter referred to as PBS (pH 5), Nissui Pharmaceutical) to give a suspension of 2×10⁹ or 3×10⁹ lactic acid bacteria/ml.

Chitosan (Chitosan 10, 80% deacetylated, 0.5% viscosity = 5 to 20 cps., Wako Pure Chemical Ind.) was suspended in 30 mL of distilled water and dissolved by adding 1 N-hydrochloric acid. The solution was then adjusted to pH 5 with 0.1 N sodium hydroxide/H₂O and made up to 100 mL with distilled water to give an aqueous solution of chitosan. Then, 10 ml of the suspension of lactic acid bacteria were mixed with 10 ml of this aqueous solution of chitosan to provide a lactic acid bacteria-containing composition.

The effectiveness of the present invention was evaluated as follows. The stomach cancer cell line MKN 45 was suspended in 10% fetal calf serum-RPMI 1640 medium at a final concentration of 5×10⁴ cells/ml and distributed into the wells of a 96-well microtiter plate. 100 µl per well. After 3 days of culture at 37 °C, the MKN 45 stomach cancer cells adherent to the microtiter plate were washed with PBS × 3 to prepare a monolayer of MKN 45. Separately, the colon cancer cell line Caco 2 was suspended in Eagle's MEM supplemented with 20% serum at a final concentration of 5×10⁴ cells/ml and distributed into the wells of a 96-well microtiter plate, 100 µl/well. After 3 days of culture at 37 °C, the Caco 2 colon cancer cells adherent to the microtiter plate were washed with PBS × 3 to prepare a monolayer of Caco 2 colon cancer cells. The suspension of lactic acid bacteria-containing composition and the suspension of lactic acid bacteria were respectively diluted 2-fold with PBS (pH 5) and 0.1 ml of each dilution was added to the MKN 45 stomach cancer cell monolayer or the Caco 2 colon cancer cell monolayer, followed by incubation at 37 °C for 10 minutes. After the cells were washed with PBS, the lactic acid bacteria were Gram-stained and the lactic acid bacteria adherent to the cells were counted under the light microscope. As a result, the lactic acid bacteria-containing composition of the present invention was found to be highly adherent to MKN 45 stomach cancer cells and Caco 2 colon cancer cells (Tables 1 and 2).

**Table 1**

| Adhesion to stomach cancer cell line MKN45 (n=4) | | | |
|---|---|---|---|
| Lactic acid bacteria | Viable count (cells/ml) | Chitosan , conc. (mg/ml) | Number of adherent cells ± SD (cells/10000 µm²) |
| *Lactobacillus salivarius* WB1004 (FEM P-15360) | 1×10⁹ | 0 | 45±10 |
| | | 1.25 | 491±78** |
| *Lactobacillus salivarius* WB1004 (FREM P-15360) | 1×10⁹ | 0 | 45±10 |
| | | 0.15 | 368±39** |
| *Lactobacillus brevis* WB1005 (FREM P-15361) | 1.5×10⁹ | 0 | 0 |
| | | 0.075 | 458±43** |
| *Lactobacillus rhamnosus* (JCM1136) | 1.5×10⁹ | 0 | 3±3 |
| | | 0.6 | 514±226** |
| *Lactobacillus paracasei* subsp*. paracasei* (CIP103918) | 1.5×10⁹ | 0 | 0 |
| | | 0.075 | 140±28** |
| *Lactobacillus casei* (CIP103137) | 1.5×10⁹ | 0 | 0 |
| | | 0.075 | 77±21** |
| *Lactobacillus plantarum* (JCM1149) | 1.5×10⁹ | 0 | 0 |
| | | 0.075 | 181±56** |
| *Lactobacillus salivarius* subsp. *salivarius* (CIP103140) | 1.5×10⁹ | 0 | 39±9 |
| | | 1.25 | 353±73** |
| *Lactobacillus salivarius* subsp. *salicinius* (JCM1150) | 1.5×10⁹ | 0 | 9±2 |
| | | 1.25 | 213±25** |
| *Lactobacillus helveticus* (JCM1120) | 1.5×10⁹ | 0 | 7±2 |
| | | 0.3 | 632±136** |
| *Lactobacillus fermentum* (JCM1173) | 1.5×10⁹ | 0 | 12±3 |
| | | 0.3 | 831±248** |
| *Lactobacillus delbrueckii* subsp. *bulgaricus* (JCM1002) | 1.5×10⁹ | 0 | 9±4 |
| | | 0.6 | 351±72** |
| *Lactobacillus delbrueckii* subsp. *lactis* (JCM1248) | 1.5×10⁹ | 0 | 6±3 |
| | | 1.25 | 509±126** |
| *Lactobacillus acidophilus* (CIP76.13) | 1.5×10⁹ | 0 | 0 |
| | | 0.3 | 56±19** |
| *Lactobacillus reuteri* (JCM1112) | 1×10⁹ | 0 | 333±61 |
| | | 0.3 | 1604±177** |
| *Lactobacillus gasseri* (JCM1131) | 1.5×10⁹ | 0 | 37±12 |
| | | 1.25 | 312±96** |
| *Lactobacillus johnsonii* (CNCM I-1225) | 1.5×10⁹ | 0 | 13±4 |
| | | 0.16 | 691±68** |
| *Lactobacillus acidophilus* WB2001 | 1.5×10⁹ | 0 | 26±18 |
| | | 0.15 | 2141±189** |

| | | | |
|---|---|---|---|
| Student's t-test**: significant difference, p<0.01 | | | |

**Table 2**

| Adhesion to Colon cancer cell line Caco 2 (n=4) | | | |
|---|---|---|---|
| Lactic acid bacteria | Viable count (cells/ml) | Chitosan , conc. (mg/ml) | Number of adherent cells ± SD (cells/10000 µm²) |
| *Lactobacillus salivarius* WB1004 (FREM P-15360) | 1×10⁹ | 0 | 104±36 |
| | | 1.25 | 475±62** |
| *Lactobacillus brevis* WB1005 (FREM P-15361) | 1.5×10⁹ | 0 | 3±2 |
| | | 0.075 | 177±75** |
| *Lactobacillus casei* (CIP103137) | 1.5×10⁹ | 0 | 3±1 |
| | | 0.075 | 71±34* |
| *Lactobacillus plantarum* (JCM1149) | 1.5×10⁹ | 0 | 2±2 |
| | | 0.075 | 324±169** |
| *Lactobacillus acidophilus* (CIP76.13) | 1.5×10⁹ | 0 | 11±4 |
| | | 0.3 | 134±58* |
| *Lactobacillus reuteri* (JCM1112) | 1×10⁹ | 0 | 65±8 |
| | | 0.6 | 1127±431** |

| | | | |
|---|---|---|---|
| Student's t-test**: significant difference, p<0.01 | | | |
| Student's t-test*: significant difference, p<0.05 | | | |

### Example 2

Lactic acid bacteria were inoculated into 1% glucose-containing GAM Bouillon (Nissui Pharmaceutical) and a liquid culture was carried out at 37 °C for 24 hours. After completion of culture, the culture fluid was centrifuged at 3000 rpm for 5 minutes to give a cellular concentrate. To this concentrate was added PBS (pH 5), whereby a suspension containing 2×10⁹ lactic acid bacteria/ml was obtained.

Then, 10 ml of the obtained suspension of lactic acid bacteria was mixed with 10 ml of the aqueous solution of chitosan prepared in the same manner as in Example 1 to give a lactic acid bacteria-containing composition.

The effect of the present invention was evaluated by the same procedure as in Example 1. As a result, the lactic acid bacteria-containing composition of the present invention was found to be highly adherent to MKN 45 stomach cancer cells and Caco 2 colon cancer cells (Tables 3 and 4).

**Table 3**

| Adhesion to stomach cancer cell line MKN45 (n=4) | | | |
|---|---|---|---|
| Lactic acid bacteria | Viable count (cells/ml) | Chitosan, conc. (mg/ml) | Number of adherent cells ± SD (cells/10000 µm²) |
| *Enterococcus faecalis* (ATCC29212) | 1×10⁹ | 0 | 0 |
| | | 0.15 | 582±114** |
| *Enterococcus faecalis* (JCM5803) | 1×10⁹ | 0 | 0 |
| | | 0.075 | 312±21** |
| *Enterococcus faecium* (JCM5804) | 1×10⁹ | 0 | 0 |
| | | 0.15 | 338±73** |

| | | | |
|---|---|---|---|
| Student's t-test**: significant difference, p<0.01 | | | |

**Table 4**

| Adhesion to colon cancer cell line Caco 2 (n=4) | | | |
|---|---|---|---|
| Lactic acid bacteria | Viable count (cells/ml) | Chitosan, conc.(mg/ml) | Number of adherent cells ± SD (cells/10000 µm²) |
| *Enterococcus faecium* (JCM5804) | 1×10⁹ | 0 | 0 |
| | | 0.15 | 314±38** |

| | | | |
|---|---|---|---|
| Student's t-test**: significant difference, p<0.01 | | | |

### Example 3

A lactic acid bacteria-containing composition was prepared by mixing 10 ml of a suspension of lactic acid bacteria prepared as in Example 1 with 10 ml of an aqueous solution of chitosan prepared as in Example 1.

The effect of the present invention was evaluated by the same procedure as in Example 1. As a result, the lactic acid bacteria-containing composition of the present invention was found to be highly adherent to MKN 45 stomach cancer cells and Caco 2 colon cancer cells (Tables 5 and 6).

**Table 5**

| Adhesion to stomach cancer cell line MKN45 (n=4) | | | |
|---|---|---|---|
| Lactic acid bacteria | Viable count (cells/ml) | Chitosan, conc.(mg/ml) | Number of adherent cells ± SD (cells/10000 µm²) |
| *Lactococcus lactis* (ATCC11454) | 1×10⁹ | 0 | 8±4 |
| | | 0.15 | 882±233** |
| *Leuconostoc mesenteroides* (JCM6124) | 1×10⁹ | 0 | 108±30 |
| | | 0.3 | 844±28** |

| | | | |
|---|---|---|---|
| Student's t-test**: significant difference, p<0.01 | | | |

**Table 6**

| Adhesion to Colon cancer cell line Caco 2 (n=4) | | | |
|---|---|---|---|
| Lactic acid bacteria | Viable count (cells/ml) | Chitosan, conc. (mg/ml) | Number of adherent cells ± SD (cells/10000 µm²) |
| *Leuconostoc mesenteroides* (JCM6124) | 1×10⁹ | 0 | 5±1 |
| | | 0.3 | 2939±563** |

| | | | |
|---|---|---|---|
| Student's t-test**: significant difference, p<0.01 | | | |

### Example 4

A lactic acid bacteria-containing composition was prepared by mixing 10 ml of a suspension of lactic acid bacteria prepared as in Example 2 with 10 ml of an aqueous solution of chitosan prepared as in Example 1.

The effect of the present invention was evaluated by the same procedure as in Example 1. As a result, the lactic acid bacteria-containing composition of the present invention was found to be highly adherent to Caco 2 colon cancer cells (Table 7).

**Table 7**

| Adhesion to Colon cancer cell line Caco 2 (n=4) | | | |
|---|---|---|---|
| Lactic acid bacteria | Viable count (cells/ml) | Chitosan, conc.(mg/ml) | Number of adherent cells ± SD (cells/10000 µm²) |
| *Bifidobacterium bifidum* WB1003 (FREM P-12612) | 1×10⁹ | 0 | 110±10 |
| | | 0.6 | 194±35** |
| *Bifidobacterium breve* (JCM1192) | 1×10⁹ | 0 | 11±16 |
| | | 0.6 | 499±34** |
| *Bifidobacterium infantis* (JCM1222) | 1×10⁹ | 0 | 0 |
| | | 0.15 | 403±61** |
| *Bifidobacterium longum* WB1001 (FREM P-12610) | 1×10⁹ | 0 | 0 |
| | | 0.3 | 362±50** |

| | | | |
|---|---|---|---|
| Student's t-test**: significant difference, p<0.01 | | | |

### Example 5

Foods for Specified Health Use "MEIJI Bulgaria Yogurt" (Meiji Milk Products) and "Onaka-e-GG!" (Takanashi Milk Products) were respectively diluted with sterilized PBS and inoculated on BL agar medium, 0.1 ml per dish. After 3 days of anaerobic culture at 37 °C, the emergent colonies were isolated. The bacterial stains thus isolated were identified by Gram-staining and API 50 CL (Japan BioMérieux Biotech). From "MEIJI Bulgaria Yogurt", *Lactobacillus delbrueckii* subsp. *bulgaricus* and *Streptococcus salivarius* subsp. *thermophilus* were isolated. From "Onaka-e-GG!", *Lactobacillus rhamnosus* was isolated. The lactic acid bacteria thus isolated and identified were cultured in liquid media as in Example 1 to give suspensions each containing 3×10⁹ lactic acid bacteria/ml.

Then, 10 ml of each suspension of lactic acid bacteria was mixed with 10 ml of an aqueous solution of chitosan prepared in the same manner as in Example 1 to give a lactic acid bacteria-containing composition.

The effect of the present invention was evaluated by the same procedure as in Example 1. As a result, the lactic acid bacteria-containing composition of the present invention was found to be highly adherent to MKN 45 stomach cancer cells and Caco 2 colon cancer cells (Tables 8 and 9).

**Table 8**

| Adhesion to stomach cancer cell line MKN45 (n=4) | | | |
|---|---|---|---|
| Lactic acid bacteria | Viable count (cells/ml) | Chitosan, conc. (mg/ml) | Number of adherent cells ± SD (cells/10000 µm²) |
| *Lactobacillus delbrueckii* subsp. *bulgaricus* (Meiji Milk Products) | 1.5×10⁹ | 0 | 6±1 |
| | | 0.6 | 164±10** |
| *Streptococcus salivarius* subsp. *thermophilus* (Meiji Milk Products) | 1.5×10⁹ | 0 | 44±26 |
| | | 0.3 | 1356±102** |
| *Lactobacillus rhamnosus* (Takanashi Milk Products) | 1.5×10⁹ | 0 | 8±3 |
| | | 0.3 | 366±43** |

| | | | |
|---|---|---|---|
| Student's t-test**: significant difference, p<0.01 | | | |

**Table 9**

| Adhesion to Colon cancer cell line Caco 2 (n=4) | | | |
|---|---|---|---|
| Lactic acid bacteria | Viable count (cells/ml) | Chitosan, conc. (mg/ml) | Number of adherent cells ± SD (cells/10000 µm²) |
| *Lactobacillus delbrueckii* subsp. *bulgaricus* (Meiji Milk Products) | 1.5×10⁹ | 0 | 7±3 |
| | | 0.15 | 1210±637** |
| *Streptococcus salivarius* subsp. *thermophilus* (Meiji Milk Products) | 1.5×10⁹ | 0 | 37±26 |
| | | 0.15 | 1159±305** |
| *Lactobacillus rhamnosus* (Takanashi Milk Products) | 1.5×10⁹ | 0 | 10±3 |
| | | 0.15 | 1797±239** |

| | | | |
|---|---|---|---|
| Student's t-test**: significant difference, p<0.01 | | | |

### Example 6

The suspension of *Lactobacillus salivarius* WB 1004 and the suspension of *Lactobacillus salivarius* WB 1004-containing composition as obtained in Example 1 were respectively lyophilized to prepare an *Lactobacillus salivarius* WB 1004 cell-containing powder and a powdered *Lactobacillus salivarius* WB 1004 cell-containing composition.

The effect of the present invention was evaluated by the same procedure as in Example 1 using the *Lactobacillus salivarius* WB 1004 cell-containing powder and the powdered *Lactobacillus salivarius* WB 1004 cell-containing composition each suspended in No. 1 solution J. P. (pH 1.2). As a result, the lactic acid bacteria-containing composition of the present invention was found to be highly adherent to MKN 45 gastric cancer cells (Table 10).

**Table 10**

| Adhesion to stomach cancer cell line MKN45 (n=4) | |
|---|---|
| Sample | Number of adherent cells ± SD (cells/10000 µm²) |
| Powdered lactic acid bacteria | 51±9 |
| Powdered lactic acid bacteria-containing composition | 230±12** |

| | |
|---|---|
| Student's t-test**: significant difference, p<0.01 | |

### Example 7

A suspension of *Lactobacillus salivarius* WB 1004 prepared in the same manner as in Example 1 (5 mL) was centrifuged at 3000 rpm for 5 minutes to obtain a cellular concentrate of *Lactobacillus salivarius* WB 1004.

Various grades of chitosan (inclusive of chitosan oligosaccharide) were respectively suspended in 30 ml of distilled water and dissolved by adding 1 N hydrochloric acid. After pH adjustment with 0.1 N-sodium hydroxide, each solution was made up to 100 ml with distilled water to give an aqueous solution of chitosan. Then, 10 ml of the aqueous solution of chitosan was added to the cellular concentrate of lactic acid bacteria to give a lactic acid bacteria-containing composition. The effect of the present invention was evaluated by the same procedure as in Example 1. As a result, the lactic acid bacteria-containing composition of the present invention was found to be highly adherent to MKN 45 stomach cancer cells and Caco 2 colon cancer cells (Tables 11 and 12).

**Table 11**

| Adhesion to stomach cancer cell line MKN45 (n=4) | | | |
|---|---|---|---|
| Chitosan | Chitosan solution (pH) | Chitosan, conc.(mg /ml) | Number of adherent cells ± SD (cells/10000 µm² |
| Chitosan 100, deacetylation 80%, 0.5% viscosity: 50 to 150 cps Wako Pure Chemical Ind. | 5 | 0 | 36±17 |
| | | 0.25 | 205±23** |
| Chitosan 100, deacetylation 80%, 0.5% viscosity: 300 to 700 cps Wako Pure Chemical Ind. | 5 | 0 | 36±17 |
| | | 0.3 | 200±17** |
| Chitosan 1000, deacetylation: 75 to 90%, 0.5% viscosity: 800 to 1300 cps Wako Pure Chemical Ind. | 5 | 0 | 36±17 |
| | | 0.25 | 190±20** |
| Koyo Chitosan, deacetylation: 46.1%, 0.5% viscosity: 35 cps Koyo Chemical | 2 | 0 | 36±17 |
| | | 0.00015 | 103±21** |
| Chitosan, deacetylation 7%, 0.5% viscosity: 430 cps Yaidzu Suisan Chemical | 5 | 0 | 36±17 |
| | | 0.75 | 648±163** |
| Kimitsu Chitosan, deacetylation 75 to 85%, 0.5% viscosity:5 to 20 cps Kimitsu Chemical | 4.5 | 0 | 36±17 |
| | | 10 | 140±37** |
| Kimitsu Chitosan, deacetylation 95%, 0.5% viscosity: 5 to 20 cps Kimitsu Chemical | 6.5 | 0 | 36±17 |
| | | 2.5 | 173±40** |
| Water-soluble chitosan, mol. wt. 3000 to 30000. Wako Pure Chemical Ind. | 4 | 0 | 30±6 |
| | | 0.63 | 717±92** |
| Chitosan Oligosaccharide, 70% di-hexasaccharides: mol. wt.20000/30% lactic acid Yaidzu Suisan Chemical | 4 | 0 | 30±6 |
| | | 0.75 | 927±166** |

| | | | |
|---|---|---|---|
| Student's t-test**: significant difference, p<0.01 | | | |

**Table 12**

| Adhesion to Colon cancer cell line Caco 2 (n=4) | | | |
|---|---|---|---|
| Chitosan | Chitosan solution (pH) | Chitosan, conc. (mg/ml) | Number of adherent cells ± SD (cells/10000 µm²) |
| Chitosan oligosaccharide, 70% di-hexasaccharides: mol. wt. 20000/30% lactic acid | 4 | 0 | 24±7 |
| | | 0.1 | 76±21** |
| Yaidzu Suisan Chemical | | | |

| | | | |
|---|---|---|---|
| Student's t-test**: significant difference, p<0.01 | | | |

### Example 8

A suspension of *Lactobacillus salivarius* WB 1004 prepared by the same procedure as in Example 1 (5 ml) was centrifuged at 3000 rpm for 5 minutes to give a cellular concentrate of *Lactobacillus salivarius* WB 1004.

Chitosan (chitosan 10, 80% acetylated, 0.5% viscosity = 5 to 20 cps, Wako Pure Chemical Ind., or Chitosan LL, 80% deacetylated, 0.5% viscosity = 20 cps, Yaidzu Suisan Chemical) was suspended in 30 ml of distilled water and solubilized with a varying kind of acid. After adjustment to pH 3 or 5 with 0.1 N-sodium hydroxide, each solution was made up to 100 ml with distilled water to give an aqueous solution of chitosan. Then, 10 ml of the chitosan solution were added to the cellular concentrate of lactic acid bacteria to give a lactic acid bacteria-containing composition.

The effect of the present invention was evaluated by the same procedure as in Example 1. As a result, the lactic acid bacteria-containing composition of the present invention was found to be highly adherent to MKN 45 stomach cancer cells and Caco 2 colon cancer cells (Table 13).

**Table 13**

| Adhesion to stomach cancer cell line MKN45 (n=4) | | | | |
|---|---|---|---|---|
| Acid for dissolving chitosan | Chitosan | Chitosan solution (pH) | Chitosan, conc. (mg/ml) | Number of adherent cells ± SD (cells/10000 µm²) |
| Acetic acid | Chitosan 10 | 3 | 0 | 36±17 |
| | | | 0.075 | 229±31** |
| Lactic acid | Chitosan 10 | 3 | 0 | 36±17 |
| | | | 0.075 | 295±70** |
| Citric acid | Chitosan 10 | 3 | 0 | 36±17 |
| | | | 0.15 | 367±26** |
| Ascorbic acid | Chitosan 10 | 3 | 0 | 36±17 |
| | | | 0.075 | 364±85** |
| Succinic acid | Chitosan 10 | 3 | 0 | 36±17 |
| | | | 0.075 | 662±144** |
| Tartaric acid | Chitosan 10 | 3 | 0 | 36±17 |
| | | | 0.075 | 346±38** |
| Malic acid | Chitosan 10 | 3 | 0 | 36±17 |
| | | | 1.25 | 353±92** |
| Aspartic acid | Chitosan LL | 5 | 0 | 29±7 |
| | | | 0.15 | 1802±162** |
| Glutamic acid | Chitosan LL | 5 | 0 | 32±2 |
| | | | 0.15 | 1811±196** |
| Adipic acid | Chitosan LL | 5 | 0 | 105±25 |
| | | | 0.15 | 1802±358** |
| Maleic acid | Chitosan LL | 5 | 0 | 105±25 |
| | | | 0.15 | 1931±134** |
| Nicotinic acid | Chitosan LL | 5 | 0 | 105±25 |
| | | | 0.15 | 1706±93** |

| | | | | |
|---|---|---|---|---|
| Student's t-test**: significant difference, p<0.01 | | | | |

### Example 9

Amoxicillin (Sigma) and vancomycin (Shionogi Pharmaceutical) were added to drinking water at final concentrations of 200 µg/ml and 10 µg/ml, respectively, and ICR mice (male, 5 weeks old) were allowed to drink the drinking water for 5 days to eliminate the bacteria in the stomach. Then, the drinking water was changed to sterile water and, after an overnight fast, a two-fold PBS dilution of the suspension of *Lactobacillus salivarius* WB 1004-containing composition or the suspension of *Lactobacillus salivarius* WB 1004 cells as prepared in Example 1, Example 17 or Example 19 was orally administered in a dose of 0.5 ml (8×10⁸ cells). After 30 minutes, the mouse was sacrificed, the stomach was isolated, and the gastric contents were removed. The stomach was washed with PBS and using a glass homogenizer, the stomach was homogenized with 9 weight parts of 0.5 M NaCl per weight part of the stomach. The homogenate thus obtained was appropriately diluted with 0.5 M NaCl and spread on dishes containing Modified LBS Agar Medium (Mitsuoka, T.: A Color Atlas of Anaerobic Bacteria, Sobun-sha). The dishes were anaerobically incubated at 37 °C for 24 hours and the number of bacterial cells adherent to the stomach was determined from the number of the colonies developed. Several of the colonies were analyzed by API 50 CH (Japan BioMérieux Biotech) and identified to be *Lactobacillus salivarius* WB 1004. As a result, the lactic acid bacteria-containing composition of the present invention is highly adherent to the mouse stomach (Table 14).

**Table 14**

| Adhesion to the mouse stomach (n=4-5) | | |
|---|---|---|
| Polymer (conc.) | Sample | Number of adherent cells ± SD (Log10CFU/stomach) |
| Chitosan 10 (0.25%) | Lactic acid bacteria-containing composition | 7.1±0.8* |
| | Lactic acid bacteria | 5.7±0.2 |
| Eudragit RL (0.5%) | Lactic acid bacteria-containing composition | 6.8±0.4** |
| | Lactic acid bacteria | 3.6±0.8 |
| Eudragit E100 (2%) | Lactic acid bacteria-containing composition | 7.3±0.6** |
| | Lactic acid bacteria | 4.2±1.0 |

| | | |
|---|---|---|
| Student's t-test*: significant difference, p<0.05 | | |
| Student's t-test**: significant difference, p<0.01 | | |

### Example 10

MKN 45 stomach cancer cells were suspended in 10% FCS-RPMI 1640 medium at a final concentration of 5×10⁴ cells/ml and distributed into the wells of a 96-well microtiter plate. 0.1 ml/well. After 3 days of culture at 37 °C, the MKN 45 stomach cancer cells adherent to the microtiter plate were washed with PBS × 3 to prepare a monolayer of MKN 45 stomach cancer cells.

The suspension of *Lactobacillus salivarius* WB 1004 or that of an *Lactobacillus salivarius* WB 1004-containing composition as obtained in Example 1 (0.1 ml) was added to the above MKN 45 monolayer and, after 30 minutes of incubation, the MKN 45 stomach cancer cells were washed with PBS × 3. Then, 0.1 ml of PBS was added and a sonic disruption was carried out using a sonicator (Model 7250, Seiko Electronics) at 20 kHz for 5 seconds to solubilize the MKN 45 stomach cancer cells and disperse the *Lactobacillus salivarius* WB 1004 adherent to the cells. Then, this sonicated fluid was diluted with PBS and spread on MRS agar plates and the viable count of *Lactobacillus salivarius* WB 1004 was determined. As a result, whereas the viable count of lactic acid bacteria adherent to MKN 45 stomach cancer cells was 10^{4.8±0.1} when the suspension of *Lactobacillus salivarius* WB 1004 cells was used, the viable count was 10^{6.3±0.1}, about 30 times as high, when the suspension of *Lactobacillus salivarius* WB 1004-containing composition of the present invention was used.

### Example 11

Polylysine (50% polylysine, Asama Kasei) was dissolved in 100 ml of PBS. Then, 10 ml of this polylysine solution were mixed with 10 ml of a suspension of lactic acid bacteria (pH 7, 2×10⁹/ml) as prepared in the same manner as Example 1 or 2 to give a lactic acid bacteria-containing composition. The effect of the present invention was evaluated in the same manner as in Example 1. However, the test for adherence to the Caco 2 colon cancer cells was carried out at pH 7 for 30 minutes. As a result, the lactic acid bacteria-containing composition of the present invention was found to be highly adherent to MKN 45 stomach cancer cells and Caco 2 colon cancer cells (Tables 15 and 16).

**Table 15**

| Adhesion to stomach cancer cell line MKN45 (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 19±8 |
| | 12.5 | 237±83** |
| *Lactobacillus brevis* WB1005 (FREM P-15361) | 0 | 20±4 |
| | 6.3 | 183±16** |
| *Lactobacillus casei* (CIP103137) | 0 | 2±1 |
| | 1.6 | 29±7** |
| *Lactobacillus plantarum* (JCM1149) | 0 | 16±1 |
| | 1.6 | 78±19** |
| *Enterococcus faecium* (JCM5804) | 0 | 10±5 |
| | 3.1 | 393±57** |
| *Lactococcus lactis* (ATCC11454) | 0 | 10±4 |
| | 1.6 | 188±51** |
| *Bifidobacterium bifidum* WB1003 (FREM P-12612) | 0 | 15±7 |
| | 3.1 | 93±24** |
| *Bifidobacterium infantis* (JCM1222) | 0 | 8±2 |
| | 1.6 | 64±16** |

| | | |
|---|---|---|
| Student's t-test**: significant difference, p<0.01 | | |

**Table 16**

| Adhesion to colon cancer cell line Caco 2 (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 9±2 |
| | 1.3 | 112±12** |
| *Lactobacillus brevis* WB1005 (FREM P-15361) | 0 | 6±1 |
| | 2.5 | 75±7** |
| *Lactobacillus acidophilus* WB2001 | 0 | 4±3 |
| | 1.3 | 160±11** |
| *Enterococcus faecium* (JCM5804) | 0 | 12±1 |
| | 2.5 | 176±18** |
| *Bifidobacterium bifidum* WB1003 (FREM P-12612) | 0 | 5±2 |
| | 0.6 | 625±32** |

| | | |
|---|---|---|
| Student's t-test**: significant difference, p<0.01 | | |

### Example 12

Cholestyramine resin (Sigma) was suspended in 100 ml of PBS. Then, 10 ml of the suspension of cholestyramine resin was mixed with 10 ml of a suspension of lactic acid bacteria (pH 7, 2×10⁹/ml) prepared in the same manner as in Example 1 or 2 to give a lactic acid bacteria-containing composition. The effect of the present invention was evaluated in the same manner as in Example 1. However, the test for adherence to the Caco 2 colon cancer cells was carried out at pH 7 for 30 minutes. As a result, the lactic acid bacteria-containing composition of the present invention was found to be highly adherent to MKN 45 stomach cancer cells and Caco 2 colon cancer cells (Tables 17 and 18).

**Table 17**

| Adhesion to stomach cancer cell line MKN45 (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 172±44 |
| | 1.3 | 644±306** |
| *Lactobacillus brevis* WB1005 (FREM P-15361) | 0 | 57±30 |
| | 0.078 | 624±77** |
| *Lactobacillus casei* (CIP103137) | 0 | 5±1 |
| | 0.31 | 264±28** |
| *Lactobacillus acidophilus* (CIP76.13) | 0 | 21±5 |
| | 1.3 | 1859±280** |
| *Enterococcus faecium* (JCM5804) | 0 | 95±18 |
| | 0.078 | 394±103** |
| *Lactococcus lactis* (ATCC11454) | 0 | 40±7 |
| | 1.3 | 1619±454** |
| *Bifidobacterium breve* (JCM1192) | 0 | 14±4 |
| | 0.078 | 53±6** |
| *Bifidobacterium infantis* (JCM1222) | 0 | 42±3 |
| | 0.078 | 627±94** |

| | | |
|---|---|---|
| Student's t-test**: significant difference, p<0.01 | | |

**Table 18**

| Adhesion to colon cancer cell line Caco 2 (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 9±2 |
| | 1.3 | 359±18** |
| *Lactobacillus brevis* WB1005 (FREM P-15361) | 0 | 6±1 |
| | 2.5 | 317±17** |
| *Lactobacillus acidophilus* (CIP76.13) | 0 | 1±1 |
| | 2.5 | 197±21** |
| *Lactobacillus johnsonii* (CNCM I-1225) | 0 | 1±1 |
| | 5.0 | 69±13** |
| *Lactobacillus acidophilus* WB2001 | 0 | 4±3 |
| | 1.3 | 324±16** |
| *Enterococcus faecium* (JCM5804) | 0 | 12±1 |
| | 2.5 | 139±14** |
| *Lactococcus lactis* (ATCC11454) | 0 | 1±1 |
| | 5.0 | 141±11** |
| *Bifidobacterium bifidum* WB1003 (FREM P-12612) | 0 | 5±2 |
| | 0.6 | 659±29** |

| | | |
|---|---|---|
| Student's t-test**: significant difference, p<0.01 | | |

### Example 13

Protamine (Asama Kasei) was dissolved in 60 ml of distilled water followed by addition and dissolution of 0.9 g of sodium chloride. The solution was adjusted to pH 4 with 1 N-HCl and diluted with distilled water to make to 100 ml, whereby an aqueous protamine solution of 2.5% or 0.15% concentration was obtained. Then, 10 ml of this aqueous solution of protamine were mixed with 10 ml of a suspension of *Lactobacillus salivarius* WB 1004 (pH 4, 3×10⁹/ml) prepared in the same manner as in Example 1 to give a lactic acid bacteria-containing composition. The effect of the present invention was evaluated by the same procedure as in Example 1. As a result, the lactic acid bacteria-containing composition of the present invention was found to be highly adherent to MKN 45 stomach cancer cells and Caco 2 colon cancer cells (Tables 19 and 20).

**Table 19**

| Adhesion to stomach cancer cell line MKN45 (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 57±13 |
| | 12.5 | 128±28** |

| | | |
|---|---|---|
| Student's t-test**: significant difference, p<0.01 | | |

**Table 20**

| Adhesion to colon cancer cell line Caco 2 (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 18±5 |
| | 0.75 | 105±33** |

| | | |
|---|---|---|
| Student's t-test**: significant difference, p<0.01 | | |

### Example 14

Protamine sulfate (salmon-derived; Wako Pure Chemical Ind.) was dissolved in 60 ml of distilled water followed by addition and dissolution of 0.9 g of sodium chloride. The solution was adjusted to pH 7 with 1 N-sodium hydroxide and diluted with distilled water to make 100 ml, whereby an aqueous protamine solution of 0.06 to 0.5% concentration was obtained. Then, 10 ml of each protamine solution were mixed with 10 ml of a suspension of lactic acid bacteria (pH 7, 3×10⁹/ml) prepared as in Example 1 or 2 to provide a lactic acid bacteria-containing composition. The effect of the present invention was evaluated by the same procedure as in Example 1. However, the test for adherence to the Caco 2 colon cancer cells was carried out at pH 7 for 30 minutes. As a result, the lactic acid bacteria-containing composition of the present invention was found to be highly adherent to Caco 2 colon cancer cells (Table 21).

**Table 21**

| Adhesion to colon cancer cell line Caco 2 (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 9±2 |
| | 0.3 | 161±22** |
| *Lactobacillus brevis* WB1005 (FREM P-15361) | 0 | 6±1 |
| | 0.6 | 412±46** |
| *Lactobacillus plantarum* (JCM1149) | 0 | 0 |
| | 1.3 | 122±17** |
| *Lactobacillus acidophilus* (CIP76.13) | 0 | 1±1 |
| | 0.6 | 44±8** |
| *Lactobacillus johnsonii* (CNCM I-1225) | 0 | 1±1 |
| | 2.5 | 22±7** |
| *Lactobacillus acidophilus* WB2001 | 0 | 4±3 |
| | 0.6 | 290±41** |
| *Enterococcus faecium* (JCM5804) | 0 | 12±1 |
| | 2.5 | 173±24** |
| *Bifidobacterium bifidum* WB1003 (FREM P-12612) | 0 | 5±2 |
| | 0.6 | 601±81** |

| | | |
|---|---|---|
| Student's t-test**: significant difference, p<0.01 | | |

### Example 15

A suspension of lactic acid bacteria (3×10⁹ cells/ml) as prepared in the same manner as in Example 1 or Example 5 (10 ml) was centrifuged at 3000 rpm for 10 minutes to give a cellular concentrate of lactic acid bacteria.

Gelatin (Type AP-100, Nitta Gelatin) was suspended in 80 ml of distilled water and dissolved by warming at 50°C, followed by spontaneous cooling. Then, 0.9 g of sodium chloride was added and dissolved and the solution was adjusted to pH 3 with 1 N-HCl and further diluted with distilled water to make 100 ml, whereby an aqueous solution of gelatin was obtained. Then, 20 ml of the gelatin solution were added to the above cellular concentrate of lactic acid bacteria to give a lactic acid bacteria-containing composition.

The effect of the present invention was evaluated by the same procedure as in Example 1. However, the test for adherence was carried out for 30 minutes. As a result, the lactic acid bacteria-containing composition of the present invention was found to be highly adherent to MKN 45 stomach cancer cells and Caco 2 colon cancer cells (Tables 22 and 23).

**Table 22**

| Adhesion to stomach cancer cell line MKN45 (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 19±5 |
| | 10 | 94±21** |
| *Lactobacillus gasseri* (JCM1131) | 0 | 28±3 |
| | 6.4 | 427±138** |
| *Lactobacillus rhamnosus* (Takanashi Milk Product) | 0 | 5±3 |
| | 6.4 | 66±21** |
| *Lactobacillus johnsonii* (CNCM I-1225) | 0 | 7±4 |
| | 2.5 | 25±10* |
| *Lactobacillus acidophilus* WB2001 | 0 | 33±8 |
| | 40 | 612±90** |

| | | |
|---|---|---|
| Student's t-test**: significant difference, p<0.01 | | |
| Student's t-test*: significant difference, p<0.05 | | |

**Table 23**

| Adhesion to colon cancer cell line Caco 2 (n=4) | | |
|---|---|---|
| Lactic acid bacteria | Polymer conc. (mg/ml) | Number of adherent cells ± SD (cells/10000 µm²) |
| *Enterococcus faecium* (JCM5804) | 0 | 157±81 |
| | 2.5 | 1400±633** |
| *Bifidobacterium bifidum* WB1003 (FREM P-12612) | 0 | 7±5 |
| | 0.6 | 55±19** |

| | | |
|---|---|---|
| Student's t-test**: significant difference, p<0.01 | | |

### Example 16

Low-molecular-weight gelatin (Miyagi Kagaku Kogyo) (10 g) was dissolved in 80 ml of distilled water followed by addition and dissolution of 0.9 g of sodium chloride. This solution was adjusted to pH 3 with 1 N-HCl and diluted with distilled water to make 100 ml, whereby an aqueous solution of low-molecular-weight gelatin was obtained. Then, 10 ml of this 10% aqueous solution of low-molecular-weight gelatin were mixed with 10 ml of a suspension of *Lactobacillus salivarius* WB 1004 (pH 3, 3×10⁹ cells/ml) to give a lactic acid bacteria-containing composition. The effect of the present invention was evaluated by the same procedure as in Example 1. However, the adherence test was carried out for 30 minutes. As a result, the lactic acid bacteria-containing composition of the present invention was found to be highly adherent to MKN 45 stomach cancer cells (Table 24).

**Table 24**

| Adhesion to stomach cancer cell line MKN45 (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 19±5 |
| | 50 | 304±79** |

| | | |
|---|---|---|
| Student's t-test**: significant difference, p<0.01 | | |

Aminoalkyl methacrylate copolymer RS (Eudragit RL100, Röhm) was suspended in 80 ml of distilled water and the suspension was heated at 80 °C with stirring for 2 hours to disperse the aminoalkyl methacrylate copolymer RS (mean particle diameter 49.3 nm). Then, 0.96 g of PBS powder (Dulbecco^{'}s PBS(-), Nissui Pharamceutical) was added and dissolved and the solution was diluted with distilled water to make 100 ml and filtered (filter: 1.2 µm) to give an aqueous dispersion of aminoalkyl methacrylate copolymer RS. Then, 10 ml of this aqueous dispersion of aminoalkyl methacrylate copolymer RS were mixed with 10 ml of a suspension of lactic acid bacteria (pH 7, 3×10⁹ cells/ml) prepared in the same manner as in Example 5 to give a lactic acid bacteria-containing composition. The affect of the present invention was evaluated by the same procedure as in Example 1 or Example 5. As a result, the lactic acid bacteria-containing composition of the present invention was found to be highly adherent to MKN 45 stomach cancer cells and Caco 2 colon cancer cells (Tables 25 and 26).

**Table 25**

| Adhesion to stomach cancer cell line MKN45 (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 30±5 |
| | 0.15 | 559±50** |
| *Lactobacillus gasseri* (JCM1131) | 0 | 2±2 |
| | 2.5 | 212±68** |
| *Lactobacillus rhamnosus* (Takanashi Milk Product) | 0 | 5±2 |
| | 0.83 | 31±9** |
| *Lactobacillus acidophilus* (CIP76.13) | 0 | 7±2 |
| | 0.28 | 13±3* |
| *Lactobacillus johnsonii* (CNCM I-1225) | 0 | 7±3 |
| | 0.031 | 20±9* |
| *Lactobacillus acidophilus* WB2001 | 0 | 13±7 |
| | 0.28 | 1139±412** |

| | | |
|---|---|---|
| Student's t-test**: significant difference, p<0.01 | | |
| Student's t-test*: significant difference, p<0.05 | | |

**Table 26**

| Adhesion to colon cancer cell line Caco 2 (n=4) | | |
|---|---|---|
| Lactic acid bacteria | Polymer conc. (mg/ml) | Number of adherent cells ± SD (cells/10000 µm²) |
| *Lactabacillus salivarius* WB1004 (FREM P-15360) | 0 | 233±107 |
| | 0.05 | 3526±484** |
| *Lactobacillus gasseri* (JCM1131) | 0 | 87±33 |
| | 0.05 | 1593±647** |
| *Lactobacillus johnsonii* (CNCM I-1225) | 0 | 27±29 |
| | 12.5 | 77±27* |
| *Lactobacillus rhamnosus* (Takanashi Milk Product) | 0 | 90±95 |
| | 0.05 | 627±385* |
| *Enterococcus faecium* (JCM5804) | 0 | 131±46 |
| | 0.05 | 2451±825** |
| *Bifidobacterium infantis* (JCM1222) | 0 | 19±18 |
| | 0.05 | 501±232** |

| | | |
|---|---|---|
| Student's t-test**: significant difference, p<0.01 | | |
| Student's t-test*: significant difference, p<0.05 | | |

### Example 18

Aminoalkyl methacrylate copolymer RS (Eudragit RS100, Röhm) was suspended in 80 ml of distilled water and the suspension was heated at 80 °C with stirring for 2 hours to disperse the aminoalkyl methacrylate copolymer RS (mean particle diameter 60.9 nm). Then, 0.9 g of sodium chloride was dissolved and the solution was adjusted to pH 4 with 1 N-HCl and diluted to 100 ml with distilled water, followed by filtration (1.2 µm), to give an aqueous dispersion of aminoalkyl methacrylate copolymer RS. Then, 10 ml of this aqueous dispersion of aminoalkyl methacrylate copolymer RS were mixed with 10 ml of a suspension of *Lactobacillus salivarius* WB 1004 (pH 4, 3×10⁹ cells/ml) prepared in the same manner as in Example 1 to give a lactic acid bacteria-containing composition. The effect of the present invention was evaluated by the same procedure as in Example 1. As a result, the lactic acid bacteria-containing composition of the present invention was found to be highly adherent to MKN 45 stomach cancer cells and Caco 2 colon cancer cells (Tables 27 and 28).

**Table 27**

| Adhesion to stomach cancer cell line MKN45 (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 57±13 |
| | 1.5 | 556±135** |

| | | |
|---|---|---|
| Student's t-test**: significant difference, p<0.01 | | |

**Table 28**

| Adhesion to colon cancer cell line Caco 2 (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 18±5 |
| | 3 | 89±35* |

| | | |
|---|---|---|
| Student's t-test*: significant difference, p<0.05 | | |

### Example 19

A suspension of lactic acid bacteria (3×10⁹ cells/ml) obtained in the same manner as in Example 1 or Example 5 (10 ml) was centrifuged at 3000 rpm for 10 minutes to give a cellular concentrate of lactic acid bacteria.

Aminoalkyl methacrylate copolymer E (Eudragit E100, Röhm) was suspended in 60 ml of distilled water and dissolved with 1 N-HCl. Then, 0.9 g of sodium chloride was added and dissolved. The solution was adjusted to pH 5 with 0.1 N-sodium hydroxide and diluted to 100 ml with distilled water to give an aqueous solution of aminoalkyl methacrylate copolymer E. Then, 20 ml of the aqueous solution of aminoalkyl methacrylate copolymer E were mixed with the above cellular concentrate of lactic acid bacteria to give a lactic acid bacteria-containing composition. The effect of the present invention was evaluated by the same procedure as in Example 1. However, the test for adherence was carried out for 30 minutes. As a result, the lactic acid bacteria-containing composition of the present invention was found to be highly adherent to MKN 45 stomach cancer cells and Caco 2 colon cancer cells (Tables 29 and 30).

**Table 29**

| Adhesion to stomach cancer cell line MKN45 (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 57±13 |
| | 25 | 140±50* |
| *Lactobacillus gasseri* (JCM1131) | 0 | 28±13 |
| | 8 | 777±197** |
| *Lactobacillus rhamnosus* (Takanashi Milk Product) | 0 | 5±3 |
| | 1.3 | 138±45** |
| *Lactobacillus acidophilus* (CIP76.13) | 0 | 14±7 |
| | 8 | 156±30** |
| *Lactobacillus johnsonii* (CNCM I-1225) | 0 | 12±3 |
| | 8 | 101±30** |
| *Lactobacillus acidophilus* WB2001 | 0 | 156±20 |
| | 3.2 | 392±81** |

| | | |
|---|---|---|
| Student's t-test**: significant difference, p<0.01 | | |
| Student^{'}s t-test*: significant difference, p<0.05 | | |

**Table 30**

| Adhesion to colon cancer cell line Caco 2 (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 18±5 |
| | 1.5 | 182±50** |
| *Lactobacillus gasseri* (JCM1131) | 0 | 126±59 |
| | 0.8 | 1516±366** |
| *Lactobacillus rhamnosus* (Takanashi Milk Product) | 0 | 65±44 |
| | 0.05 | 431±151** |
| *Enterococcus faecium* (JCM5804) | 0 | 87±79 |
| | 0.05 | 961±284** |
| *Bifidobacterium bifidum* WB1003 (FREM P-12612) | 0 | 142±90 |
| | 0.8 | 414±91** |
| *Bifidobacterium infantis* (JCM1222) | 0 | 5±4 |
| | 0.8 | 231±64** |

| | | |
|---|---|---|
| Student's t-test**: significant difference, p<0.01 | | |

### Example 20

Lactoferrin (Wako Pure Chemical Ind.) (5 g) was dissolved in 60 mL of distilled water followed by addition and dissolution of 0.9 g of sodium chloride. This solution was adjusted to pH 4 with 1 N-HCl, diluted with distilled water to 100 mL, and filtered (1.2 µm to give an aqueous solution of lactoferrin. Then, 10 ml of this 5% aqueous solution of lactoferrin were mixed with 10 ml of a suspension of *Lactobacillus salivarius* WB 1004 (pH 4, 3×10⁹ cells/ml) prepared in the same manner as in Example 1 to give a lactic acid bacteria-containing composition. The effect of the present invention was evaluated by the same procedure as in Example 1. As a result, the lactic acid bacteria-containing composition of the present invention was found to be highly adherent to MKN 45 stomach cancer cells (Table 31).

**Table 31**

| Adhesion to stomach cancer cell line MKN45 (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 57±13 |
| | 25 | 333±71** |

| | | |
|---|---|---|
| Student's t-test**: significant difference, p<0.01 | | |

### Example 21

Egg white lysozyme (Wako Pure Chemical Ind.) was dissolved in 60 ml of distilled water followed by addition and dissolution of 0.9 g of sodium chloride. This solution was adjusted to pH 4 with 1 N-HCl and diluted to 100 ml with distilled water to give an aqueous solution of lysozyme. Then, 10 ml of this lysozyme solution were mixed with 10 ml of a suspension of *Lactobacillus salivarius* WB 1004 (pH 4, 3×10⁹ cells/ml) prepared in the same manner as in Example 1 to give a lactic acid bacteria-containing composition. The effect of the present invention was evaluated by the same procedure as in Example 1. As a result, the lactic acid bacteria-containing composition of the present invention was found to be highly adherent to MKN 45 stomach cancer cells and Caco 2 colon cancer cells (Tables 32 and 33).

**Table 32**

| Adhesion to stomach cancer cell line MKN45 (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 30±6 |
| | 25 | 174±42** |

| | | |
|---|---|---|
| Student's t-test**: significant difference, p<0.01 | | |

**Table 33**

| Adhesion to colon cancer cell line Caco 2 (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 24±7 |
| | 6.3 | 47±7* |

| | | |
|---|---|---|
| Student's t-test*: significant difference. p<0.05 | | |

### Example 22

A suspension of lactic acid bacteria (3×10⁹ cells/ml) prepared in the same manner as in Example 1 (1 ml) was centrifuged at 3000 rpm for 5 minutes to give a cellular concentrate of lactic acid bacteria. To this concentrate was added 1 ml of a mixture of the aqueous chitosan solution, aqueous gelatin solution, Eudragit RL100 suspension and aqueous lactoferrin solution each prepared in the same manner as in Example 1, Example 15, Example 17 and Example 20 to give a lactic acid bacteria-containing composition.

The effect of the present invention was evaluated by the same procedure as in Example 1. As a result, the lactic acid bacteria-containing composition of the present invention was found to be highly adherent to MKN 45 stomach cancer cells (Table 34).

**Table 34**

| Adhesion to stomach cancer cell line MKN45 (n=4) | | | |
|---|---|---|---|
| Lactic acid bacteria | Polymer | Polymer conc. (mg/ml) | Number of adherent cells ± SD (cells/10000 µm²) |
| *Lactobacillus salivarius* WB1004 (FREM P-15360) | None | 0 | 61±7 |
| | Eudragit RL100 | 0.2 | 422±44** |
| | Gelatin AP100 | 10 | |
| *Lactobacillus salivarius* WB1004 (FREM P-15360) | None | 0 | 46±12 |
| | Gelatin AP100 | 10 | 368±73** |
| | Bovine | 25 | |
| | lactoferrin | | |
| *Lactobacillus salivarius* WB1004 (FREM P-15360) | None | 0 | 46±12 |
| | Chitosan 10 | 1.25 | 645±190** |
| | Bovine | 25 | |
| | lactoferrin | | |
| *Lactobacillus gasseri* (JCM1131) | None | 0 | 51±24 |
| | Chitosan 10 | 2.5 | 251±70** |
| | Lactoferrin | 12.5 | |
| *Lactobacillus gasseri* (JCM1131) | None | 0 | 51±24 |
| | Gelatin AP100 | 10 | 230±35** |
| | Lactoferrin | 6.25 | |

| | | | |
|---|---|---|---|
| Student's t-test**: significant difference, p<0.01 | | | |

### Example 23

A culture of lactic acid bacteria (3×10⁹ cells/ml) as obtained in the same manner as in Example 1 or Example 5 was centrifuged at 3000 rpm for 5 minutes to give a cellular concentrate of lactic acid bacteria. Then, PBS was added to the obtained cellular concentrate to prepare a suspension containing 3×10⁹ lactic acid bacteria/ml.

Chitosan (Chitosan 10) was suspended in 30 ml of distilled water and dissolved with 1 N-HCl. The solution was adjusted to pH 5 with 0.1 N-NaOH and diluted to 100 ml with distilled water to give an aqueous solution of chitosan. Then, 1 ml of the chitosan solution was mixed with 1 ml of the suspension of lactic acid bacteria to give a lactic acid bacteria-containing composition.

The effect of the present invention was evaluated as follows. THP-1 monocytic leukemia cells were suspended in PBS at a final concentration of 6×10⁶ cells/ml and the suspension was distributed into the wells of a 96-well microtiter plate, 50 µl/well.

A suspension (50 µl) of the lactic acid bacteria-containing composition or a suspension of lactic acid bacteria, each as diluted 2-fold with PBS, was added to the THP-1 monocytic leukemia cells in the wells and the plate was incubated at 37 °C for 30 minutes. Then, 15% sucrose-containing PBS was distributed into the wells of a 96-well microtiter plate, 200 µl/well, and 20 µl of the reaction mixture of the incubated (30 min.) THP-1 monocytic leukemia cells with the suspension of lactic acid bacteria-containing composition or the suspension of lactic acid bacteria were layered and the plate was centrifuged at 500 rpm for 5 minutes to recover a cellular residue. This residue was washed with 200 µl of PBS and recentrifuged at 500 rpm for 5 minutes to give a cell pellet. The lactic acid bacteria were Gram-stained and those lactic acid bacteria adherent to the cells were counted under the light microscope. As a result, the lactic acid bacteria-containing composition of the present invention was found to be highly adherent to the THP-1 monocytic leukemia cells (Table 35).

**Table 35**

| Adhesion to monocytic leukemia cell line THP-1 (n=4) | | |
|---|---|---|
| Lactic acid bacteria | Polymer conc. (mg/ml) | Number of adherent cells ± SD (cells/cell) |
| *Lactobacillus gasseri* (JCM1131) | 0 | 5±4 |
| | 5.0 | 42±4** |
| *Lactobacillus johnsonii* (CNCM I-1225) | 0 | 4±1 |
| | 1.3 | 22±7** |
| *Lactobacillus rhamnosus* (Takanashi Milk Product) | 0 | 1±1 |
| | 5.0 | 16±12* |
| *Lactobacillus plantarum* (JCM1149) | 0 | 1±1 |
| | 5.0 | 17±6** |
| *Enterococcus faecium* (JCM5804) | 0 | 1±2 |
| | 5.0 | 22±5** |
| *Bifidobacterium infantis* (JCM1222) | 0 | 1±1 |
| | 5.0 | 22±10** |

| | | |
|---|---|---|
| Student's t-test*: significant difference, p<0.05 | | |
| Student's t-test**: significant difference, p<0.01 | | |

### Example 24

A lactic acid bacteria-containing composition was prepared by mixing 1 ml of a suspension of lactic acid bacteria as obtained in the same manner as in Example 23 with 1 ml of an aqueous dispersion of aminoalkyl methacrylate copolymer RS as prepared in the same manner as in Example 17. The effect of the present invention was evaluated by the same procedure as in Example 23. As a result, the lactic acid bacteria-containing composition of the present invention was found to be highly adherent to THP-1 monocytic leukemia cells (Table 36).

**Table 36**

| Adhesion to monocytic leukemia cell line THP-1 (n=4) | | |
|---|---|---|
| Lactic acid bacteria | Polymer conc. (mg/ml) | Number of adherent cells ± SD (cells/cell) |
| *Lactobacillus gasseri* (JCM1131) | 0 | 1±1 |
| | 0.2 | 24±18* |
| *Lactobacillus johnsonii* (CNCM I-1225) | 0 | 1±0.2 |
| | 0.8 | 17±6** |
| *Lactobacillus rhamnosus* (Takanashi Milk Product) | 0 | 0.3±0.3 |
| | 0.2 | 22±8** |
| *Lactobacillus plantarum* (JCM1149) | 0 | 0.2±0.2 |
| | 0.05 | 7±1** |
| *Enterococous faecium* (JCM5804) | 0 | 14±17 |
| | 0.2 | 88±44** |
| *Bifidobacterium infantis* (JCM1222) | 0 | 1±1 |
| | 0.8 | 32±16** |

| | | |
|---|---|---|
| Student's t-test*: significant difference, p<0.05 | | |
| Student's t-test**: significant difference. p<0.01 | | |

### Example 25

A lactic acid bacteria-containing composition was prepared by mixing 1 ml of a suspension of lactic acid bacteria as prepared in the same manner as in Example 23 with 1 ml of an aqueous dispersion of aminoalkyl methacrylate copolymer E as prepared in the same manner as in Example 19.

The effect of the present invention was evaluated as follows. THP-1 monocytic leukemia cells were suspended in saline (pH 5) at a final concentration of 6×10⁶ cells/ml and distributed into the wells of a 96-well microtiter plate, 50 µl per well.

A suspension of the lactic acid bacteria-containing composition or a suspension of lactic acid bacteria, each as diluted 2-fold with saline (pH 4), was added, 50 µl/well, to the THP-1 monocytic leukemia cells in advance distributed into the wells of a microtiter plate and the plate was incubated at 37 °C for 60 minutes. Separately, 15% sucrose-PBS was distributed into the wells of a 96-well microtiter plate, 200 µl per well, and 20 µl of a reaction mixture consisting of THP-1 monocytic leukemia cells and said suspension of lactic acid bacteria-containing composition or suspension of lactic acid bacteria, as incubated for 60 minutes, were layered and the plate was centrifuged at 500 rpm for 5 minutes to obtain a cellular concentrate. The obtained concentrate was washed with 200 µl of PBS and recentrifuged at 500 rpm for 5 minutes to obtain a pellet. The lactic acid bacteria were Gram-stained and the lactic acid bacteria adherent to the cells were counted under the light microscope. As a result, the lactic acid bacteria-containing composition of the present invention was found to be highly adherent to THP-1 monocytic leukemia cells (Table 37).

**Table 37**

| Adhesion to monocytic leukemia cell line THP-1 (n=4) | | |
|---|---|---|
| Lactic acid bacteria | Polymer conc. (mg/ml) | Number of adherent cells ± SD (cells/cell) |
| *Lactobacillus gasseri* (JCM1131) | 0 | 7±8 |
| | 0.05 | 104±14** |
| *Lactobacillus johnsonii* (CNCM I-1225) | 0 | 10±6 |
| | 0.2 | 49±21** |
| *Lactobacillus rhamnosus* (Takanashi Milk Product) | 0 | 9±10 |
| | 0.05 | 129±10** |
| *Lactobacillus plantarum* (JCM1149) | 0 | 3±3 |
| | 3.2 | 25±3** |
| *Enterococcus faecium* (JCM5804) | 0 | 2±4 |
| | 0.2 | 138±42** |
| *Bifidobacterium infantis* (JCM1222) | 0 | 1±1 |
| | 0.2 | 22±11** |

| | | |
|---|---|---|
| Student's t-test*: significant difference, p<0.05 | | |
| Student's t-test**: significant difference, p<0.01 | | |

### Example 26

A culture of lactic acid bacteria (3×10⁹ cells/ml) as obtained by the same procedure as in Example 23 was centrifuged at 3000 rpm for 5 minutes to give a cellular concentrate. To the obtained concentrate was added a sufficient amount of saline (pH 4) to give a suspension containing 3×10⁹ lactic acid bacteria/ml.

Gelatin (Type AP-100, Nitta Gelatin) was suspended in 80 ml of distilled water and dissolved by warming at 50 °C. After spontaneous cooling. 0.9 g of sodium chloride was added and dissolved and the solution was adjusted to pH 4 with 1 N-HCl and diluted to 100 ml with distilled water to give an aqueous solution of gelatin. Then, 1 ml of the suspension of lactic acid bacteria (in saline, pH 4) was mixed with 1 ml of the above aqueous solution of gelatin to give a lactic acid bacteria-containing composition.

The effect of the present invention was evaluated as follows. THP-1 monocytic leukemia cells were suspended in saline (pH 4) at a final concentration of 6×10⁶ cells/ml and distributed into the wells of a 96-well microtiter plate, 50 µl/well.

A suspension of lactic acid bacteria-containing composition or a suspension of lactic acid bacteria, each as diluted 2-fold with saline (pH 4), was added, 50 µl per well, to the THP-1 monocytic leukemia cells in the wells at a microtiter plate and the plate was incubated at 37 °C for 90 minutes. Separately, 15% sucrose-PBS was distributed into the wells of a 96-well microtiter plate, 200 µl per well, and 20 µl of a reaction mixture of said suspension of lactic acid bacterial-containing composition or suspension of lactic acid bacteria with THP-1 monocytic leukemia cells, as incubated for 90 minutes, were layered and the plate was centrifuged at 500 rpm for 5 minutes to obtain a cellular concentrate. The concentrate thus obtained was washed with 200 µl of PBS and recentrifuged at 500 rpm for 5 minutes to give a cell pellet. The lactic acid bacteria were Gram-stained and those lactic acid bacteria adherent to the leukemia cells were counted under the light microscope. As a result, the lactic acid bacteria-containing composition of the present invention was found to be highly adherent to THP-1 monocytic leukemia cells (Table 38).

**Table 38**

| Adhesion to monocytic leukemia cell line THP-1 (n=4) | | |
|---|---|---|
| Lactic acid bacteria | Polymer conc. (mg/ml) | Number of adherent cells ± SD (cells/cell) |
| *Lactobacillus gasseri* (JCM1131) | 0 | 1±1 |
| | 0.32 | 5±1** |
| *Lactobacillus johnsonii* (CNCM I-1225) | 0 | 2±2 |
| | 1.3 | 12±9 |
| *Lactobacillus rhamnosus* (Takanashi Milk Product) | 0 | 0.2±0.1 |
| | 0.32 | 10±3** |
| *Lactobacillus plantarum* (JCM1149) | 0 | 1±0.3 |
| | 0.08 | 2±1* |
| *Enterococcus faecium* (JCM5804) | 0 | 1±2 |
| | 5.0 | 16±9* |
| *Bifidobacterium infantis* (JCM1222) | 0 | 1±1 |
| | 5.0 | 11±13* |

| | | |
|---|---|---|
| Student's t-test*: significant difference, p<0.05 | | |
| Student's t-test**: significant difference. p<0.01 | | |

### Example 27

To investigate the pharmacologic action-potentiating effect of the lactic acid bacteria-containing composition of the present invention, with attention paid to lactic acid which is one of pharmacoactive substances, the amount of lactic acid produced by the lactic acid bacteria adherent to the cells was determined by the following method. Thus, the lactic acid bacteria (*Lactobacillus salivarius* WB 1004 or *Enterococcus* faecium JCM 5804) containing compositions obtained in Examples 1, 12, 14, 15, 17 and 19 were respectively caused to adhere to MKN 45 stomach cancer cells by the procedure described in Example 1. After each system was washed with PBS × 3, 0.1 ml of Hanks balanced salt solution (Gibco; hereinafter referred to as HBSS) was added, followed by incubation at 37 °C for 3 hours. The lactic acid produced in the supernatant was methylated with methanolic sulfuric acid, extracted with chloroform, and assayed by gas chromatography. As a result, the lactic acid bacteria-containing composition adherent to cells gave high lactic acid production (Table 39).

**Table 39**

| Lactic acid production by the lactic acid bacteria adherent to stomach cancer cell line MKN45 | | | |
|---|---|---|---|
| Lactic acid bacteria | Polymer | Conc. (mg/ml) | Production of lactic acid (mM) |
| *Lactobacillus salivarius* (WB1004) | Chitosan 10 | 0 | 0.13 |
| | | 0.64 | 2.74 |
| | Eudragit RL | 0 | 0.13 |
| | | 0.04 | 1.15 |
| | Eudragit E100 | 0 | 0.13 |
| | | 0.3 | 0.87 |
| | Gelatin AP100 | 0 | 0.15 |
| | | 1.25 | 0.88 |
| | Cholestyramine resin | 0 | 0.15 |
| | | 0.6 | 1.79 |
| | Protamine sulfate | 0 | 0.15 |
| | | 0.3 | 1.05 |
| *Enterococcus faecium (*JCM5804) | Gelatin AP100 | 0 | 0 |
| | | 0.3 | 0.27 |

### Example 28

To investigate the pharmacologic action-potentiating effect of the lactic acid bacteria-containing composition of the present invention, with attention paid to lactic acid which is a pharmacoactive substance, the amount of lactic acid produced by the lactic acid bacteria adherent to the cells was determined by the following method. Thus, the lactic acid bacteria (*Lactobacillus salivarius* WB 1004)-containing compositions prepared in Examples 1, 15, 17 and 19 were respectively caused to adhere to Caco 2 colon cancer cells by the same procedure as described in Example 1. After each system was washed with PBS × 3, 0.1 ml of HBSS was added, and the mixture was incubated at 37 °C for 3 hours. The lactic acid in the supernatant was methylated with methanolic sulfuric acid, extracted with chloroform, and assayed by gas chromatography. As a result, the lactic acid bacteria-containing composition adherent to cells gave high lactic acid production (Table 40).

**Table 40**

| Lactic acid production by the lactic acid bacteria adherent to colon cancer cell line Caco 2 | | |
|---|---|---|
| Polymer | Conc. (mg/ml) | Production of lactic acid (mM) |
| Chitosan 10 | 0 | 0.13 |
| | 0.3 | 0.83 |
| Eudragit RL | 0 | 0.13 |
| | 0.02 | 0.54 |
| Eudragit E100 | 0 | 0.13 |
| | 0.08 | 0.62 |
| Gelatin AP100 | 0 | 0.14 |
| | 0.6 | 0.33 |

### Example 29

A water-soluble chitosan derivative (CHGA, Tokyo Kaseihin) was dissolved in distilled water. The solution was adjusted to pH 5 with 0.5 N-sodium hydroxide and diluted to 100 ml with distilled water to give an aqueous solution of water-soluble chitosan derivative. Then, 10 ml of this aqueous solution of water-soluble chitosan derivative were mixed with 10 ml of a suspension of *Lactobacillus salivarius* WB 1004 prepared by the same method as described in Example 1 to give a lactic acid bacteria-containing composition. The effect of the present invention was evaluated by the same procedure as in Example 1. As a result, the lactic acid bacteria-containing composition of the present invention was found to be highly adherent to MKN 45 stomach cancer cells (Table 41).

**Table 41**

| Adhesion to stomach cancer cell line MKN45 (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 55±10 |
| | 2.5 | 1038±172** |

| | | |
|---|---|---|
| Student's t-test**: significant difference, p<0.01 | | |

### Example 30

Chitosan oligomers (chitosan tetramer, chitosan pentamer and chitosan hexamer; Seikagaku Kogyo) were respectively dissolved in distilled water. Each solution was adjusted to pH 7 with 1 N-sodium hydroxide and diluted to 1 ml with distilled water to give an aqueous chitosan oligomer solution. Then. 0.5 ml of this aqueous chitosan oligomer solution was mixed with 0.5 ml of a suspension of *Lactobacillus salivarius* WB 1004 (pH 7, 2×10⁹ cells/ml) WB 1004 prepared by the same procedure as in Example 1 to give a lactic acid bacteria-containing composition. The effect of the present invention was evaluated by the same procedure as in Example 1 (incubation time: 30 minutes). As a result, the lactic acid bacteria-containing composition was found to be highly adsorbent to MKN 45 stomach cancer cells (Table 42).

**Table 42**

| Adhesion to stomach cancer cell line MKN45 (n=4) | | |
|---|---|---|
| Sample | Polymer conc. (mg/ml) | Number of adherent cells ± SD (cells/10000 µm²) |
| Chitosan tetramer | 0 | 190±27 |
| | 12.5 | 324±101* |
| Chitosan pentamer | 0 | 190±27 |
| | 25 | 444±51** |
| Chitosan hexamer | 0 | 190±27 |
| | 25 | 1058±161** |

| | | |
|---|---|---|
| Student's t-test*: significant difference, p<0.05 | | |
| Student's t-test**: significant difference, p<0.01 | | |

### Example 31

A suspension of lactic acid bacteria (3×10⁹ cells/ml) was prepared by the procedure described in Example 1 or Example Example 5.

Chitosan (Chitosan 10, 80% deacetylated, 0.5% viscosity = 5 to 20 cps, Wako Pure Chemical Ind.) was suspended in 50 ml of distilled water and dissolved by adding 1 N-HCl. The solution was adjusted to pH 5 with 0.1 N-sodium hydroxide and diluted to 100 ml with distilled water to provide an aqueous chitosan solution. Then, a cellular concentrate of lactic acid bacteria (1.5×10⁹ cells) as obtained by centrifugation of a suspension of lactic acid bacteria at 3000 rpm for 10 minutes was mixed with 1 ml of the aqueous chitosan solution to give a lactic acid bacteria-containing composition.

The effect of the present invention was evaluated as follows. HeLa uterine cancer cells were suspended in 10% serum-Eagles MEM at a final concentration of 5×10⁴ cells/ml and distributed into the wells of a 96-well microtiter plate, 100 µl/well. After 3 days of culture at 37 °C, the uterine cancer HeLa cells adherent to the microtiter plate were washed with PBS × 3 to prepare a monolayer of HeLa cells. Then, 0.1 ml of the suspension of lactic acid bacteria-containing composition or the suspension of lactic acid bacteria, as diluted 2-fold with PBS (pH 5), was added to the HeLa cell monolayer and after 10 minutes of incubation at 37 °C, the cells were washed with PBS. The lactic acid bacteria were Gram-stained and those lactic acid bacteria adherent to the cells were counted under the light microscope. As a result, the lactic acid bacteria-containing composition of the present invention was found to be highly adherent to uterine cancer HeLa cells (Table 43).

**Table 43**

| Adhesion to uterine cancer cell line HeLa (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 17±6 |
| | 0.5 | 601±168** |
| *Lactobacillus salivarius* subsp. *salivarius* (CIP103140) | 0 | 17±3 |
| | 0.5 | 554±50** |
| *Lactobacillus salivarius* subsp. *salicinius* (JCM1150) | 0 | 9±2 |
| | 0.25 | 1790±335** |
| *Lactobacillus acidophilus* (CIP76.13) | 0 | 23±8 |
| | 0.5 | 395±79** |
| *Lactobacillus brevis* WB1005 (FREM P-15361) | 0 | 32±5 |
| | 0.25 | 355±74** |
| *Lactobacillus casei* (CIP103137) | 0 | 3±2 |
| | 2.5 | 330±108** |
| *Lactobacillus helveticus* (JCM1120) | 0 | 47±16 |
| | 0.3 | 632±171** |
| *Lactobacillus plantarum* (JCM1149) | 0 | 11±3 |
| | 0.6 | 657±211** |
| *Lactobacillus paracasei* subsp. *paracasei* (CIP103918) | 0 | 4±2 |
| | 0.6 | 1824±361** |
| *Lactobacillus rhamnosus* (JCM1136) | 0 | 6±3 |
| | 0.6 | 756±50** |
| *Lactobacillus fermentum* (JM1173) | 0 | 1±1 |
| | 0.3 | 1486±414** |
| *Lactobacillus delbrueckii* subsp. *lactis* (JCM1248) | 0 | 3±2 |
| | 0.15 | 1301±364** |
| *Lactobacillus delbrueckii* subsp. *bulgaricus* (JCM1002) | 0 | 2±1 |
| | 1.25 | 447±36** |
| L*actobacillus delbrueckii* subsp. *bulgaricus* (Meiji Milk Products) | 0 | 6±4 |
| | 0.6 | 295±40** |
| *Lactobacillus johnsonii* (CNCM I-1225) | 0 | 3±1 |
| | 0.6 | 590±102** |
| *Lactobacillus gasseri* (JCM1131) | 0 | 14±5 |
| | 0.15 | 1567±415** |
| *Lactobacillus acidophilus* WB2001 | 0 | 18±10 |
| | 0.15 | 1308±343** |

| | | |
|---|---|---|
| Student's t-test**: significant difference, p<0.01 | | |

### Example 32

A lactic acid bacteria-containing composition was prepared by mixing 1 ml of a dispersion of aminoalkyl methacrylate copolymer RS prepared by the procedure described in Example 17 with 1 ml of a suspension of lactic acid bacteria (3×10⁹ cells/mL) obtained by the procedure described in Example 31. The effect of the present invention was evaluated in the same manner as in Example 31. As a result, the lactic acid bacteria-containing composition of the present invention was found to be highly adherent to uterine cancer HeLa cells (Table 44).

**Table 44**

| Adhesion to uterine cancer cell line HeLa (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 7±2 |
| | 0.16 | 1736±427** |
| *Lactobacillus salivarius* subsp. salivarius (CIP103140) | 0 | 17±3 |
| | 0.5 | 563±90** |
| *Lactobacillus salivarius* subsp. *salicinius* (JCM1150) | 0 | 9±2 |
| | 0.125 | 352±79** |
| *Lactobacillus acidophilus* (CIP76.13) | 0 | 23±8 |
| | 0.5 | 177±25** |
| *Lactobacillus brevis* WB1005 (FREM P-15361) | 0 | 32±5 |
| | 0.125 | 323±59** |
| *Lactobacillus casei* (CIP103137) | 0 | 3±2 |
| | 2.5 | 38±9** |
| *Lactobacillus helveticus* (JCM1120) | 0 | 47±16 |
| | 0.28 | 150±21** |
| *LactobacilluS plantarum* (JCM1149) | 0 | 11±3 |
| | 0.28 | 654±195** |
| *Lactobacillus paracasei* subsp. *paracasei* (CIP103918) | 0 | 4±2 |
| | 0.09 | 503±127** |
| *Lactobacillus rhamnosus* (JCM1136) | 0 | 6±3 |
| | 0.8 | 331±79** |
| *Lactobacillus fermentum* (JM1173) | 0 | 1±1 |
| | 0.8 | 253±27** |
| *Lactobacillus delbrueckii* subsp. *lactis* (JCM1248) | 0 | 3±2 |
| | 2.5 | 438±64** |
| *Lactobacillus delbrueckii* subsp. *bulgaricus* (JCM1002) | 0 | 2±1 |
| | 0.28 | 324±94** |
| *Lactobacillus delbrueckii* subsp*. bulgaricus* (Meiji Milk Products) | 0 | 6±4 |
| | 0.03 | 70±23** |
| *Lactobacillus johnsonii* (CNCM I-1225) | 0 | 3±1 |
| | 0.28 | 171±16** |
| *Lactobacillus gasseri* (JCM1131) | 0 | 19±8 |
| | 0.28 | 653±158** |
| *Lactobacillus acidophilus* WB2001 | 0 | 7±1 |
| | 0.28 | 312±51** |
| *Lactobacillus rhamnosus* (Takanashi Milk Products) | 0 | 4±1 |
| | 0.28 | 66±11** |

| | | |
|---|---|---|
| Student's t-test**: significant difference, p<0.01 | | |

### Example 33

The lactic acid bacteria grown by liquid culture as in Example 1 or Example 5 were suspended in PBS at a final concentration of 3×10⁹ cells/ml.

To a cellular concentrate (1.5×10⁹ cells) obtained by centrifuging the above suspension of lactic acid bacteria at 3000 rpm for 10 minutes was added 1 ml of an aqueous solution of aminoalkyl methacrylate copolymer E prepared in the same manner as in Example 19 to give a lactic acid bacteria-containing composition. The effect of the present invention was evaluated by the same procedure as in Example 31. As a result, the lactic acid bacteria-containing composition of the present invention was found to be highly adherent to uterine cancer HeLa cells (Table 45).

**Table 45**

| Adhesion to uterine, cancer cell line HeLa (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 71±18 |
| | 20 | 1566±284** |
| *Lactobacillus acidophilus* (CIP76.13) | 0 | 3±3 |
| | 3.2 | 10±3* |
| *Lactobacillus johnsonii* (CNCM I-1225) | 0 | 3±2 |
| | 8 | 19±4** |
| *Lactobacillus gasseri* (JCM1131) | 0 | 16±9 |
| | 3.2 | 492±117** |
| *Lactobacillus acidophilus* WB2001 | 0 | 12±2 |
| | 3.2 | 76±12* |
| *Lactobacillus rhamnosus* (Takanashi Milk Products) | 0 | 9±4 |
| | 8 | 25±12* |

| | | |
|---|---|---|
| Student's t-test**: significant difference, p<0.01 | | |
| Student's t-test*: significant difference, p.<0.05 | | |

### Example 34

The lactic acid bacteria grown by liquid culture as in Example 1 were suspended in PBS at a final concentration of 3×10⁹ cells/ml.

To a cellular concentrate (1.5 ×10⁹ cells) obtained by centrifuging the suspension of lactic acid bacteria at 3000 rpm for 10 minutes was added 1 ml of an aqueous solution of gelatin prepared in the same manner as in Example 15 to give a lactic acid bacteria-containing composition. The effect of the present invention was evaluated by the same procedure as in Example 31. As a result, the lactic acid bacteria-containing composition of the present invention was found to be highly adherent to uterine cancer HeLa cells (Table 46).

**Table 46**

| Adhesion to uterine cancer cell line HeLa (n=4) | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 3±2 |
| | 16 | 169±42** |
| *Lactobacillus salivarius* subsp. *Salivarius* (CIP103140) | 0 | 11±6 |
| | 6.4 | 259±84** |
| *Lactobacillus salivarius* subsp. *Salicinius* (JCM1150) | 0 | 8±5 |
| | 6.4 | 314±105** |
| *Lactobacillus acidophilus* (CIP76.13) | 0 | 3±1 |
| | 1.0 | 20±5** |
| *Lactobacillus casei* (CIP103137) | 0 | 4±2 |
| | 16 | 11±4* |
| *Lactobacillus helveticus* (JCM1120) | 0 | 47±7 |
| | 1.0 | 75±14* |
| *Lactobacillus plantarum* (JCM1149) | 0 | 13±8 |
| | 16 | 117±40** |
| *Lactobacillus paracasei* subsp. *Paracasei* (CIP103918) | 0 | 6±2 |
| | 1.0 | 33±9** |
| *Lactobacillus rhamnosus* (JCM1136) | 0 | 10±4 |
| | 16 | 150±15** |
| *Lactobacillus delbrueckii* subsp. *Lactis* (JCM1248) | 0 | 28±7 |
| | 6.4 | 428±101** |
| *Lactobacillus delbrueckii* subsp. *Bulgaricus* (JCM1002) | 0 | 1±1 |
| | 40 | 29±9** |
| *Lactobacillus johnsonii* (CNCM I-1225) | 0 | 3±2 |
| | 16 | 26±8** |
| *Lactobacillus gasseri* (JCM1131) | 0 | 92±9 |
| | 1.0 | 236±33** |
| *Lactobacilius acidophilus* WB2001 | 0 | 12±2 |
| | 2.5 | 44±13* |

| | | |
|---|---|---|
| Student's t-test**: significant difference, p<0.01 | | |
| Student's t-test*: significant difference, p<0.05 | | |

### Example 35

The lactic acid bacteria grown by liquid culture as in Example 1 or Example 5 were suspended in PBS at a final concentration of 3×10⁹ cells/ml.

Gelatin (Type LCP, Nitta Gelatin) was dissolved in 80 ml of distilled water followed by addition and dissolution of 0.9 g of sodium chloride. This solution was adjusted to pH 3 with 1 N-HCl and diluted to 100 ml with distilled water to give an aqueous solution of gelatin. A lactic acid bacteria-containing composition was prepared by adding 1 ml of the aqueous gelatin solution to a cellular concentrate (1.5×10⁹ cells) obtained by centrifuging the suspension of lactic acid bacteria at 3000 rpm for 10 minutes. The effect of the present invention was evaluated by the same procedure as in Example 31. As a result, the lactic acid bacteria-containing composition of the present invention was found to be highly adherent to uterine cancer HeLa cells (Table 47).

**Table 47**

| Lactic acid bacteria-containing composition | | |
|---|---|---|
| Lactic acid bacteria | Polymer conc. (mg/ml) | Number of adherent cells ± SD (cells/10000 µm²) |
| *Lactobacillus salivarius* WB1004 (FREM P-15360) | 0 | 20±11 |
| | 2.5 | 72±16** |
| *Lactobacillus acidophilus* (CIP76.13) | 0 | 8±5 |
| | 16 | 24±9* |
| *Lactobacillus gasseri* (JCM1131) | 0 | 16±9 |
| | 6.4 | 139±25** |
| *Lactobacillus rhamnosus* (Takanashi Milk Products) | 0 | 9±4 |
| | 1 | 25±12* |

| | | |
|---|---|---|
| Student's t-test**: significant difference. p<0.01 | | |
| Student's t-test*: significant difference, p<0.05 | | |

### Example 36

To investigate the pharmacologic action-potentiating effect of the lactic acid bacteria-containing composition of the present invention, with attention paid to lactic acid which is one of pharmacoactive substances, the amount of lactic acid produced by lactic acid bacteria adherent to cells was determined by the following procedure. The lactic acid bacteria (*Lactobacillus salivarius* WB 1004 or *Enterococcus faecium* JCM 5804)-containing compositions obtained in the same manner in Examples 31 to 35 were respectively caused to adhere to the uterine cancer HeLa cells in the same manner in Example 31. After each system was washed with PBS × 3, 01 ml of HBSS was added and the mixture was incubated at 37 °C for 3 hours. The lactic acid produced in the supernatant was methylated with methanolic sulfuric acid, extracted with chloroform, and assayed by gas chromatography. As a result, the lactic acid bacteria-containing composition adherent to cells was found to give high lactic acid production (Table 48).

**Table 48**

| Lactic acid production by the lactic acid bacteria adherent to uterine cancer cell line HeLa | | | |
|---|---|---|---|
| Lactic acid bacteria | Polymer | Conc. (mg/ml) | Production of lactic acid (mM) |
| *Lactobacillus salivarius* (WB1004) | Chitosan 10 | 0 | 0.21 |
| | | 0.6 | 0.70 |
| | Eudragit RL100 | 0 | 0.21 |
| | | 0.04 | 0.65 |
| | Eudragit E100 | 0 | 0.21 |
| | | 0.08 | 0.46 |
| | Gelatin AP100 | 0 | 0.13 |
| | | 1.25 | 0.80 |
| *Enterococcus faecium* (JCM5804) | Gelatin AP100 | 0 | 0 |
| | | 0.3 | 0.27 |

### Test Example 1

### Assay of the inhibitory effect of a Laotobacillus salivarius WB 1004-containing composition on the presence of Helicobacter pylori on the stomach cancer cell surface

### 1) Preparation of a monolayer of human stomach cancer cell line MKN 45

Human stomach cancer MKN 45 cells were suspended in 10% FCS-PRMI 1640 medium at a final concentration of 5×10⁴ cells/ml and the suspension was distributed into the wells of a 96-well microtiter plate. 01 ml per well. After 3 days of culture at 37 °C, the cells adherent to the microtiter plate were washed with PBS × 3 to prepare a monolayer.

### 2) Culture of Lactobacillus salivarius WB 1004 and preparation of a cell suspension and a lactic acid bacteria-containing composition

*Lactobacillus salivarius* WB 1004 was inoculated into MRS Broth and a liquid culture was carried out at 37 °C for 16 hours. After completion of culture, the culture fluid was centrifuged at 3000 rpm for 5 minutes to give a cellular concentrate. To the obtained cellular concentrate was added PBS to prepare a cell suspension containing 3×10⁹/ml of *Lactobacillus salivarius* WB 1004. A portion of this cell suspension was taken and mixed with an equal part of a solution composed of Chitosan 10 (0.25% aqueous solution), Eudragit RL (0.008% aqueous suspension) and Eudragit E100 (0.008% aqueous solution) to give a lactic acid bacteria-containing composition.

### 3) Culture of Helicobacter pylori and preparation of a cell suspension

*Helicobacter pylori* ATCC 43504 was microaerobically cultured in 5% horse defibrinated blood-containing brain-heart infusion (BHI) agar medium (Difco) at 37 °C for 4 days. After completion of culture, an appropriate amount of cells was harvested using a platinum loop, washed with HBSS, and suspended in HBSS at a concentration giving a turbidity (540 nm) of 5 (ca 5×10⁸ cells/ml).

### 4) Assay of the inhibitory effect on the presence of Helicobacter pylori on the cell surface

To the monolayer of MKN 45 cells was added 100 µl of a suspension of *Lactobacillus salivarius* WB 1004 or a suspension of the lactic acid bacteria-containing composition (1.6×10⁹ cells/ml for both) to cause adhesion at 37 °C for 1 hour. PBS was used as control. After the cells were washed with PBS × 3, 50 µl of a suspension of *Helicobacter pylori* were added and the mixture was incubated at 37 °C for 2 to 4 hours. The MKN 45 cell monolayer was washed with PBS × 3 and the viable count of *Helicobacter pylori* on the cell surface was determined by the following procedure.

Thus, 50 µl of 0.5% trypsin (Gibco) was caused to act on the cell monolayer at 37 °C for 2 minutes to loosen the cells from the microtiter plate. Then. 100 µl of 5% FCS-BHI liquid medium (Difco) was added so as to inactive trypsin. After appropriate dilution with a diluent, the suspension was spread on a 5% horse defibrinated blood-containing BHI agar plate and the number of cells present per cell monolayer per well was determined. To suppress growth of *Lactobacillus salivarius* WB 1004, 5 µg/ml trimethoprim and 10 µg/ml vancomycin were added to the medium and a microaerobic culture was carried out at 37 °C for 3 days.

As a result, the suspension of lactic acid bacteria-containing composition was found to significantly inhibit the presence of *Helicobacter pylori* on the stomach cancer cell surface. On the other hand, the suspension of *Lactobacillus salivarius* WB 1004 caused little inhibition (Table 49).

**Table 49**

| Inhibition of the presence of *Helicobacter pylori* on the stomach cancer cell surface by lactic acid bacteria-containing compositions | |
|---|---|
| Sample (polymer) | The rate of *Helicobacter pylori* on stomach cancer cell surface (%) |
| Lactic acid bacteria-containing composition (chitosan) | <1 |
| Lactic acid bacteria-containing composition (Eudragit RL) | 4 |
| Lactic acid bacteria-containing composition (Eudragit E100) | 15 |
| Lactic acid bacteria | 80 |
| Control | 100 |

### Test Example 2

### Assay of the inhibitory effect of a Lactobacillus salivarius WB 1004-containing composition on the presence of Escherichia coli on the colon cancer cell surface

### 1) Preparation of a monolayer of human colon cancer Caco 2 cells

Human stomach cancer MKN45 cells were suspended in 20% FCS-Eagle's MEM Broth at a final concentration of 5×10⁴ cells/ml and the suspension was distributed into the wells of a 96-well microtiter plate, 0.1 ml per well. After 3 days of culture at 37 °C, the cells adherent to the microtiter plate were washed with PBS × 3 to prepare a monolayer.

### 2) Culture of Lactobacillus salivarius WB 1004 and preparation of a cell suspension and, a lactic acid bacteria-containing composition

*Lactobacillus salivarius* WB 1004 was inoculated into MRS Broth and a liquid culture was carried out at 37 °C for 16 hours. After completion of culture, the culture fluid was centrifuged at 3000 rpm for 5 minutes to give a cellular concentrate. To the obtained cellular concentrate was added PBS to prepare a cell suspension containing 3×10⁹/ml of *Lactobacillus salivarius* WB 1004. A portion of the cell suspension was taken and mixed with an equal part of a 0.25% solution of Chitosan 10 to give a lactic acid bacteria-containing composition.

### 3) Culture of Escherichia coli and preparation of a cell suspension

*Escherichia coli* ATCC 25922 was cultured aerobically in BHI liquid medium at 30 °C for 6 hours. After completion of culture, the culture fluid was centrifuged at 3000 rpm for 5 minutes to give a cellular concentrate. To the obtained cellular concentrate was added MRS Broth to prepare an *Escherichia coli* ATCC 25922 suspension containing about 6×10⁵ cells/ml.

### 4) Assay of the inhibitory effect on the presence on the cell surface

To the monolayer of Caco 2 cells were added 100 µl of a suspension of *Lactobacillus salivarius* WB 1004 or a suspension of the lactic acid bacteria-containing composition (1.6×10⁹ cells/ml for both) to cause adhesion at 37 °C for 30 minutes. PBS was used as control. After the cells were washed with PBS × 3, 100 µl of a suspension of *Escherichia coli* was added and the mixture was incubated at 37 °C for 3 hours. The Caco 2 monolayer was washed with PBS × 3 and the viable count of *Escherichia coli* present on the cell surface was determined by the following procedure. Thus, 50 µl of 0.5% trypsin was caused to act on the cell monolayer at 37 °C for 2 minutes to loosen the cells from the microtiter plate. Then, 100 µl of BHI liquid medium were added so as to inactive trypsin. After appropriate dilution with a diluent, the suspension was spread on DHI agar plates (Nissui Pharmaceutical) and the number of cells present on the cell monolayer per well was determined.

As a result, the suspension of lactic acid bacteria-containing composition was found to significantly inhibit the presence of *Escherichia coli* on the colon cancer cell surface (Table 50).

**Table 50**

| Inhibition of the presence of *Escherichia coli* on the colon cancer cell surface by lactic acid bacteria-containing composition | |
|---|---|
| Sample (polymer) | The rate of Escherichia coli present on colon cancer cell surface (%) |
| Lactic acid bacteria-containing composition (chitosan) | 12 |
| Lactic acid bacteria | 65 |
| Control | 100 |

### Formulation Example 1

To 100 ml of a *Lactobacillus salivarius* WB 1004-containing composition prepared as in Example 1, 50 g of sucrose, 1 g of ascorbic acid and flavor were added to give a syrup.

### Formulation Example 2

To 100 ml of a *Lactobacillus salivarius* WB 1004-containing composition prepared as in Example 1, a solution composed of 50 g sucrose, 1 g ascorbic acid and 10 g gelatin and flavor was added, followed by chilling, to give a jelly.

### Formulation Example 3

A *Lactobacillus salivarius* WB 1004-containing composition prepared as in Example 1 (100 ml) was lyophilized and the resulting powder was mixed with 100 g of a commercial yogurt (MEIJI Bulgaria Yogurt, Meiji Milk Products) to give a *Lactobacillus salivarius* WB 1004-containing composition-yogurt food.

### Formulation Example 4

A *Lactobacillus salivarius* WB 1004-containing composition prepared as in Example 1 (100 ml) was lyophilized and the resulting powder was filled into an aluminum packet to give a *Lactobacillus sulivarius* WB 1004 composition to accompany a commercial yogurt (MEIJI Bulgaria Yogurt, Meiji Milk Products) for ingestion after extemporaneous mixing.

### Formulation Example 5

A *Lactobacillus salivarius* WE 1004-containing composition prepared as in Example 1 (100 ml) was lyophilized and after addition of 100 g of lactose, 10 g of corn starch, 10 g of hydroxypropylcellulose and 5 g of hydrogenated oil to the resulting powder, the mixture was compressed with a tablet machine to give tablets each weighing 240 mg.

### Formulation Example 6

A *Lactobacillus salivarius* WB 1004-containing composition prepared as in Example 1 (100 ml) was lyophilized and after addition of 100 g of lactose, log of corn starch and 10 g of hydroxypropylcellulose to the resulting powder, the mixture was granulated and dried by means of a fluidized-bed granulating machine, followed by size-selection with a 30-mesh sieve, to give fine granules.

### Formulation Example 7

A *Lactobacillus salivarius* WB 1004-containing composition prepared as in Example 1 (100 ml) was lyophilized and the resulting powder was mixed with 5 g of liquid paraffin. Separately, 75 g of white petrolatum were melted in advance and added in small portions to the above mixture. With the vessel being externally cooled, the whole mixture was caused to solidify under agitation to give ointment type suppositories.

### Formulation Example 8

A *Lactobacillus salivarius* WB 1004-containing composition prepared as in Example 1 (100 ml) was lyophilized and the resulting powder was kneaded with 9 g of macrogol 1500 and 2 g of macrogol 4000. After gradual cooling, the kneaded mass was divided in 10 and molded in suppository molds to give suppositories.

### Formulation Example 9

A *Lactobacillus salivarius* WB 1004-containing composition prepared as in Example 1 (100 ml) was lyophilized and after addition of 100 g of lactose, 10 g of corn starch and 5 g of magnesium stearate to the resulting powder, the mixture was compressed with a tablet machine to give tablet type suppositories.

### INDUSTRIAL APPLICABILITY

The present invention, constituted as above, is capable of potentiating the pharmacologic action of lactic acid bacteria showing pharmacologic action in the digestive tract and/or the urogenital organ and antagonizing the clearance of the lactic acid bacteria from the digestive tract and/or the urogenital organ. Therefore, the present invention contributes a great deal to effective elimination of *Helicobacter pylori*, elimination of enterohemorrhagic *Escherichia coli* and therapy of food allergies and enables provision of pharmaceutical products and foods for the therapy and/or prevention of urogenital infections.

## Claims

1. A lactic acid bacteria-containing composition comprising lactic acid bacteria showing pharmacologic action in the digestive tract and/or the urogenital organ
wherein the adhesion of said lactic acid bacteria to the digestive tract and/or the urogenital organ is enhanced to potentiate said pharmacologic action.

2. The lactic acid bacteria-containing composition according to Claim 1
wherein formulation of a macromolecular substance causes to enhance said adhesion of said lactic acid bacteria to the digestive tract and/or the urogenital organ.

3. The lactic acid bacteria-containing composition according to Claim 2
wherein said macromolecular substance is a basic polymer.

4. The lactic acid bacteria-containing composition according to Claim 3
wherein the basic polymer is chitosan.

5. The lactic acid bacteria-containing composition according to Claim 3
wherein the basic polymer is polylysine.

6. The lactic acid bacteria-containing composition according to Claim 3
wherein the basic polymer is cholestyramine resin.

7. The lactic acid bacteria-containing composition according to Claim 3
wherein the basic polymer is protamine.

8. The lactic acid bacteria-containing composition according to Claim 3
wherein the basic polymer is gelatin.

9. The lactic acid bacteria-containing composition according to Claim 3
wherein the basic polymer is aminoalkyl methacrylate copolymer E.

10. The lactic acid bacteria-containing composition according to Claim 3
wherein the basic polymer is aminoalkyl methacrylate copolymer RS.

11. The lactic acid bacteria-containing composition according to Claim 3
wherein the basic polymer is lactoferrin.

12. The lactic acid bacteria-containing composition according to Claim 3
wherein the basic polymer is lysozyme.

13. The lactic acid bacteria-containing composition according to Claim 1, 2, 3, 4, 5, 6, 7, 8, 9, 10, 11 or 12
wherein said pharmacologic action is elimination of *Helicobacter pylori*.

14. The lactic acid bacteria-containing composition according to Claim 1, 2, 3, 4, 5, 6, 7, 8, 9, 10, 11 or 12
wherein said pharmacologic action is elimination of enterohemorrhagic *Escherichia coli*.

15. The lactic acid bacteria-containing composition according to Claim 1, 2, 3, 4, 5, 6, 7, 8, 9, 10, 11 or 12
wherein said pharmacologic action is therapeutic activity against food allergies.

16. The lactic acid bacteria-containing composition according to Claim 1, 2, 3, 4, 5, 6, 7, 8, 9, 10, 11 or 12
wherein said pharmacologic action is therapeutic and/or prophylactic activity against infections of the urogenital organ.

17. The lactic acid bacteria-containing composition according to Claim 16
wherein the urogenital organ is the vagina.

18. A pharmaceutical product
which comprises the lactic acid bacteria-containing composition according to Claim 1, 2, 3, 4, 5, 6, 7, 8, 9, 10, 11, 12, 13, 14, 15, 16 or 17.

19. A food
which comprises the lactic acid bacteria-containing composition according to Claim 1, 2, 3, 4, 5, 6, 7, 8, 9, 10, 11, 12, 13, 14 or 15.
